# EUROPEAN PATENT APPLICATION

(11) **EP 4 342 903 A1**
(43) Date of publication of application: **27.03.2024**
(21) Application number: 23771009.0
(22) Date of filing: 03.03.2023
(51) Int. Cl.: C07F 19/00, C07F 11/00, C07C 2/34, B01J 31/34

(54) **LIGAND COMPOUND, ORGANIC CHROME COMPOUND, AND CATALYST COMPOSITION COMPRISING SAME**

(30) Priority: 14.03.2022 KR 20220031621
(71) Applicant: LG Chem, Ltd., Seoul 07336 (KR)
(72) Inventor: SA, Seok Pil, Daejeon 34122 (KR); KIM, Seok Sun, Daejeon 34122 (KR); KIM, Tae Hee, Daejeon 34122 (KR); JUNG, Seung Hwan, Daejeon 34122 (KR); SHIN, Eun Ji, Daejeon 34122 (KR); CHO, Yeon Ho, Daejeon 34122 (KR); LIM, Won Taeck, Daejeon 34122 (KR); KIM, Hee Jeong, Daejeon 34122 (KR)
(74) Representative: Hoffmann Eitle
(86) International application number: PCT/KR2023/002972
(87) International publication number: WO 2023/177130

(57) **Abstract**

The present invention relates to a novel structured ligand compound, an organic chrome compound, a catalyst composition comprising the organic chrome compound, and an ethylene oligomerization method using same, wherein the ligand compound exhibits high selectivity for 1-hexene and 1-octene while showing high catalytic activity, and thus can perform ethylene oligomerization with excellent efficiency.

## Description

### TECHNICAL FIELD

### [Cross-reference to Related Applications]

This application claims the benefit of Korean Patent Application No. 10-2022-0031621, filed on March 14, 2022, in the Korean Intellectual Property Office, the contents of which are incorporated herein by reference.

### [Technical Field]

The present invention relates to a ligand compound, an organochromium compound, a catalyst composition comprising the organochromium compound, and a method for oligomerizing ethylene using the same.

### BACKGROUND ART

A linear alpha-olefin like 1-hexene and 1-octene is used as a detergent, a lubricant, a plasticizer, or the like, particularly, used mainly as a comonomer for adjusting the density of a polymer during preparing a linear low-density polyethylene (LLDPE).

In the preparation process of the conventional linear low-density polyethylene (LLDPE), in order to adjust the density by forming a branch on a polymer backbone, the copolymerization of ethylene together with a comonomer like an alpha-olefin such as 1-hexene and 1-octene has been performed.

Accordingly, in order to prepare the LLDPE with the high comonomer content, there were defects of the cost of the comonomer accounted for a big part of the manufacturing cost. To solve such defects, various attempts have been conducted.

The linear alpha-olefin is mainly produced through a shell higher olefin process. However, according to the method, alpha-olefins with various lengths are synthesized simultaneously according to Schultz-Flory distribution, and a separate separation process is inconveniently required for obtaining a specific alpha-olefin.

To solve such defects, a method of selectively synthesizing 1-hexene through the trimerization reaction of ethylene, or selectively synthesizing 1-octene through the tetramerization reaction of ethylene has been suggested. In addition, more studies on a catalyst system enabling such selective oligomerization of ethylene have been conducted.

### [Prior Art Document]

### [Patent Document]

(Patent Document 1) US 5064802 B2

### DETAILED DESCRIPTION OF THE INVENTION

### TECHNICAL PROBLEM

A task to solve in the present invention is to provide a ligand compound having a novel structure, which shows high catalyst activity and high selectivity to 1-hexene and 1-octene, and may oligomerize ethylene in an excellent efficiency, an organochromium compound and a catalyst composition comprising the same.

### TECHNICAL SOLUTION

To solve the above tasks, the present invention provides a ligand compound, an organochromium compound, a catalyst composition and a method for oligomerizing ethylene.
(1) The present invention provides a ligand compound represented by Formula 1.

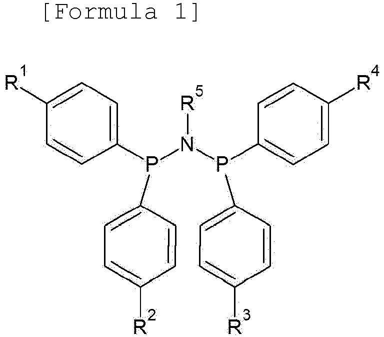

In Formula 1, R¹ to R⁴ are each independently a trialkylsilyl group, where the alkyl group is an alkyl group of 1 to 4 carbon atoms, and R⁵ is a substituent represented by any one among Formulae 2 to 4.

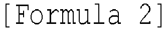

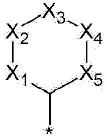

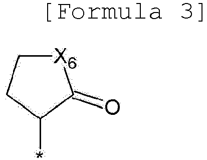

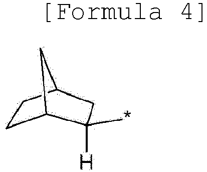

In Formulae 2 to 4, X₁ to X₆ are each independently - (CR⁶R⁷)ₙ-, -O- or -S-, where all are not -O- or -S-simultaneously, and n is an integer selected from 0 and 1 to 3, R⁶ and R⁷ are each independently hydrogen, an alkyl group of 1 to 5 carbon atoms, a trifluoroalkyl group of 1 to 5 carbon atoms, an aryl group of 6 to 10 carbon atoms, or -NR⁸R⁹, R⁸ and R⁹ are each independently hydrogen or an alkyl group of 1 to 5 carbon atoms, and * is a part bonded to N of Formula 1.

(2) The present invention provides the ligand compound according to (1), wherein R¹ to R⁴ are each independently a tripropylsilyl group or a tributylsilyl group.

(3) The present invention provides the ligand compound according to (1) or (2), wherein R⁵ is a substituent represented by any one among Formulae 2 to 4 above, X₁, X₂ and X₅ are each independently - (CR⁶R⁷)ₙ-, X₃ and X₄ are each independently - (CR⁶R⁷)ₙ- or -O-, where X₃ and X₄ are not -O-simultaneously, X₆ is -S-, R⁶ and R⁷ are each independently hydrogen, a trifluoroalkyl group of 1 to 5 carbon atoms, an aryl group of 6 to 10 carbon atoms, or -NR⁸R⁹, and R⁸ and R⁹ are each independently hydrogen or an alkyl group of 1 to 5 carbon atoms.

(4) The present invention provides the ligand compound according to any one among (1) to (3), wherein R⁵ is a substituent represented by Formula 2 above, X₁ and X₅ are - CH₂-, X₂ to X₄ are each independently -(CR⁶R⁷)ₙ-, -O- or -S-, where all are not -O- or -S- simultaneously, R⁶ and R⁷ are each independently hydrogen, a trifluoroalkyl group of 1 to 5 carbon atoms, an aryl group of 6 to 10 carbon atoms, or -NR⁸R⁹, and R⁸ and R⁹ are each independently hydrogen or an alkyl group of 1 to 3 carbon atoms.

(5) The present invention provides the ligand compound according to any one among (1) to (4), wherein the ligand compound represented by Formula 1 is one selected from the group consisting of the ligand compounds represented by Formula 2-1 to Formula 2-14.

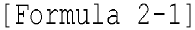

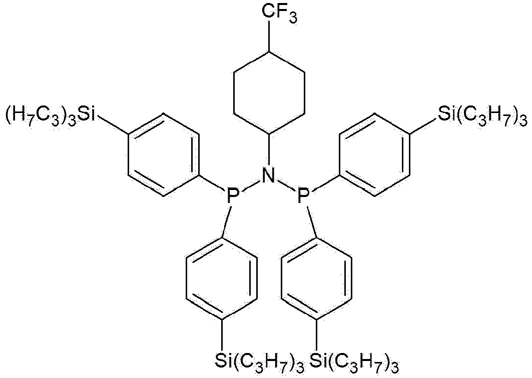

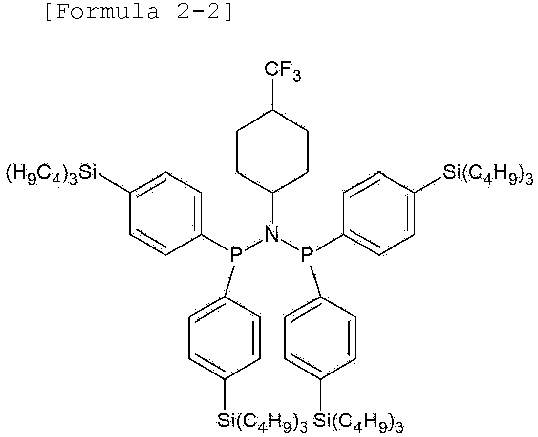

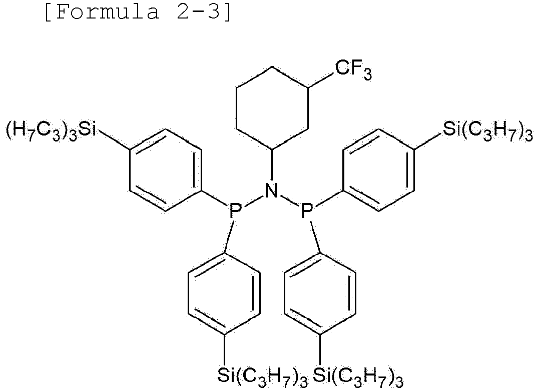

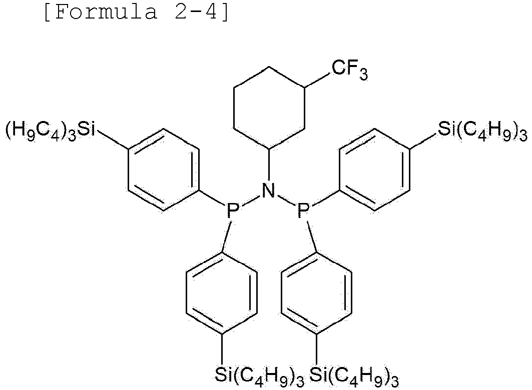

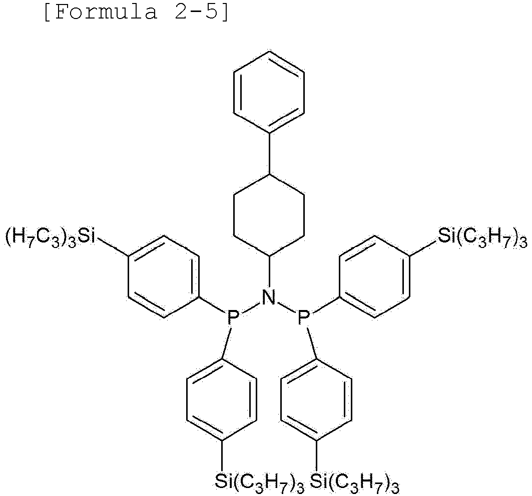

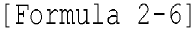

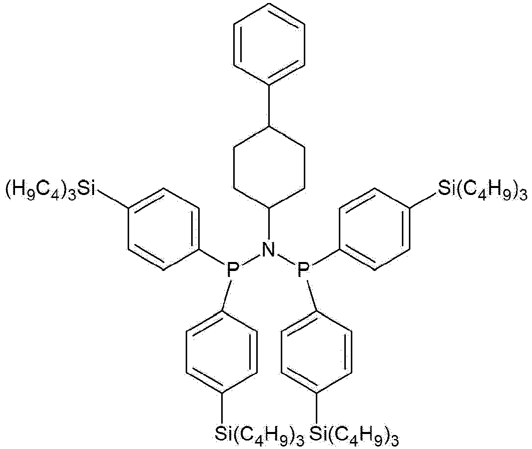

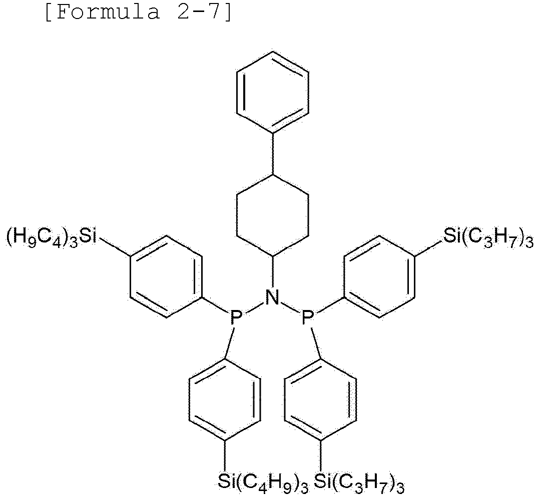

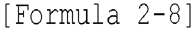

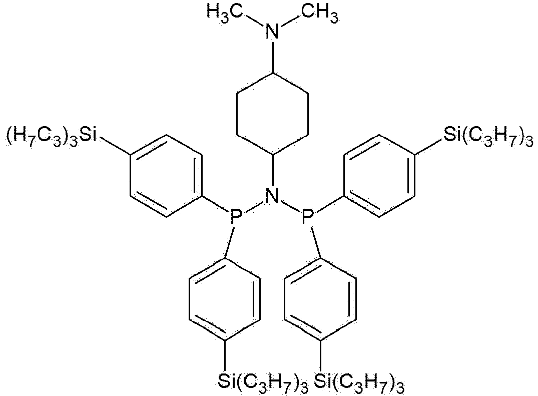

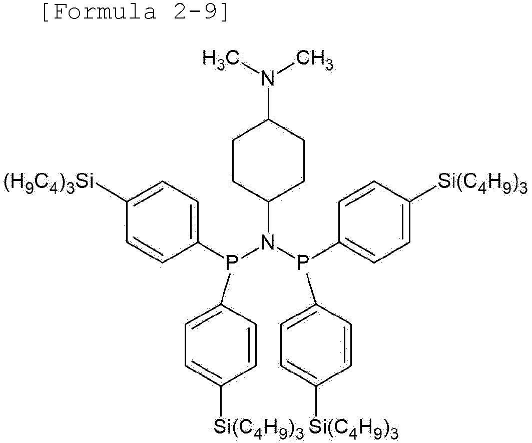

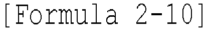

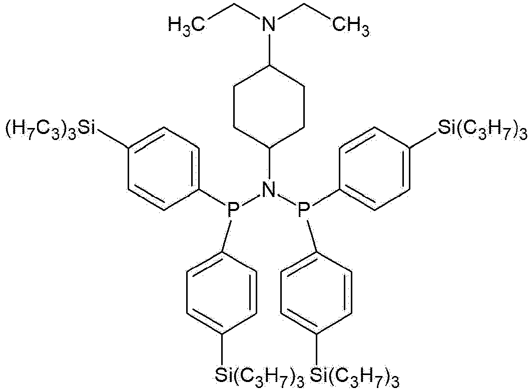

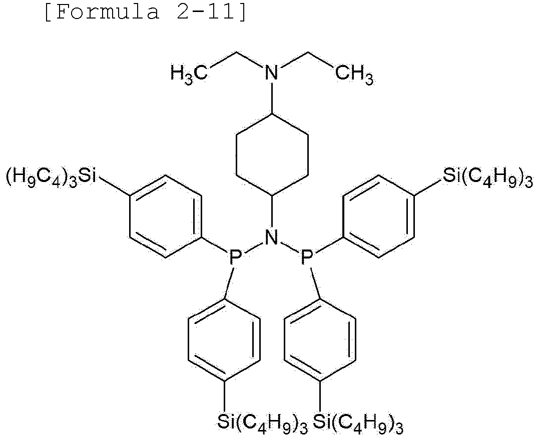

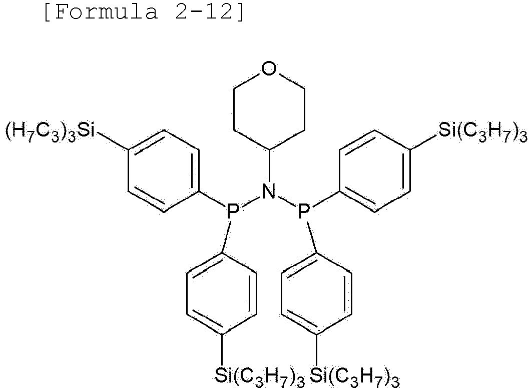

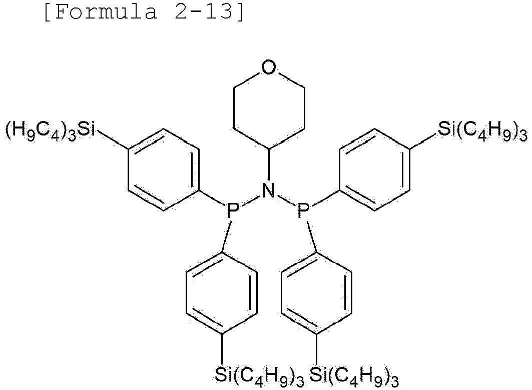

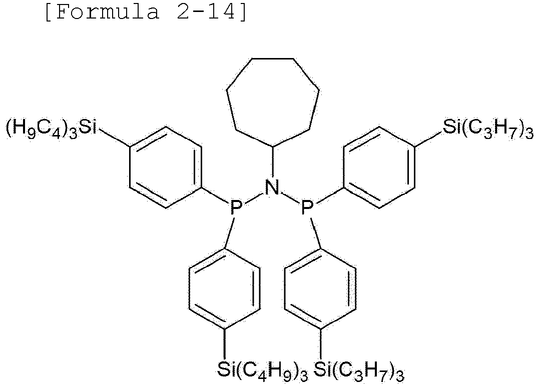

(6) The present invention provides the ligand compound according to any one among (1) to (3), wherein R⁵ is a substituent represented by Formula 3, and X₆ is -O- or -S-.

(7) The present invention provides the ligand compound according to any one among (1) to (3) and (6), wherein the ligand compound represented by Formula 1 is represented by Formula 3-1 or Formula 3-2.

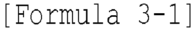

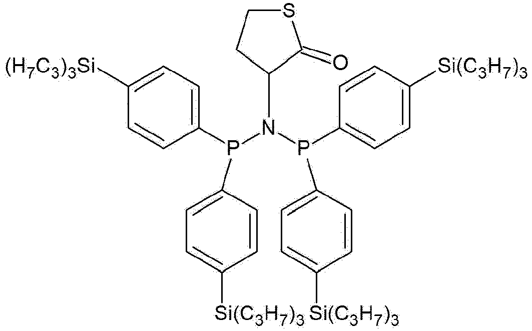

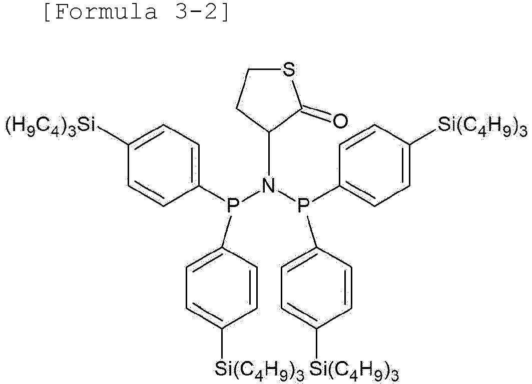

(8) The present invention provides the ligand compound according to any one among (1) to (3), wherein the ligand compound represented by Formula 1 is represented by Formula 4-1 or Formula 4-2.

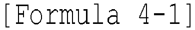

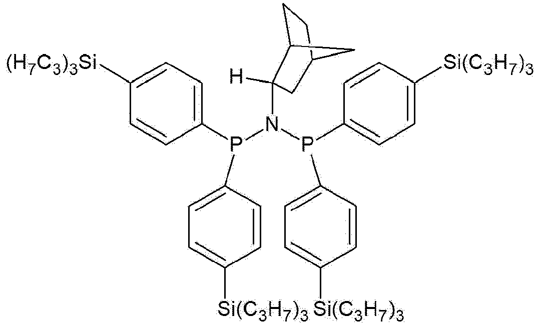

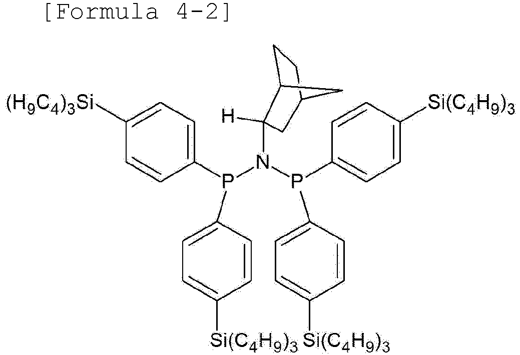

(9) The present invention provides an organochromium compound comprising the ligand compound according to any one among (1) to (8) and chromium making a coordination bond with the ligand compound.

(10) The present invention provides the organochromium compound according to (9), wherein unshared electron pairs of one or more among N and two P in the ligand compound represented by Formula 1 make coordination bonds with the chromium.

(11) The present invention provides a catalyst composition comprising the ligand compound according to any one among (1) to (8), chromium and a cocatalyst.

(12) The present invention provides the catalyst composition according to (11), wherein the chromium is derived from a chromium source, and the chromium source comprises one or more selected from the group consisting of chromium(III) acetylacetonate, chromium(III) chloride tetrahydrofuran, chromium(III) 2-ethylhexanoate, chromium(III) acetate, chromium(III) butyrate, chromium(III) pentanoate, chromium(III) laurate, chromium(III) tris(2,2,6,6-tetramethyl-3,5-heptanedionate) and chromium(III) stearate.

(13) The present invention provides the catalyst composition according to (11) or (12), wherein the cocatalyst is one or more selected from the group consisting of the compounds represented by Formula 5 to Formula 8.

[Formula 5] -[Al(R¹⁰)-O]ₐ-

In Formula 5, each R¹⁰ is independently a halogen group, a hydrocarbyl group of 1 to 20 carbon atoms, or a hydrocarbyl group of 1 to 20 carbon atoms, which is substituted with a halogen group, and a is an integer of 2 or more.

[Formula 6] E(R¹¹)₃

In Formula 6, E is aluminum or boron, and each R¹¹ is independently hydrogen, a halogen group, a hydrocarbyl group of 1 to 20 carbon atoms, or a hydrocarbyl group of 1 to 20 carbon atoms, which is substituted with a halogen group.

[Formula 7] [L⁻H]⁺[G(Y)₄]-

[Formula 8] [L]⁺[G(Y)₄]⁻

In Formula 7 and Formula 8, L is a neutral or cationic Lewis acid, [L-H]⁺ is a bronsted acid, G is an element in group 13, and each Y is independently a substituted or unsubstituted alkyl group of 1 to 20 carbon atoms or a substituted or unsubstituted aryl group of 6 to 20 carbon atoms, where if the alkyl group or the aryl group is substituted, the substituent is a halogen group, a hydrocarbyl group of 1 to 20 carbon atoms, an alkoxy group of 1 to 20 carbon atoms, or an aryloxy group of 6 to 20 carbon atoms.

(14) The present invention provides a method for preparing a linear alpha-olefin, the method comprising a step of oligomerizing ethylene in the presence of the catalyst composition according to any one among (11) to (13) (S10).

(15) The present invention provides the method for preparing a linear alpha-olefin according to (14), wherein the linear alpha-olefin is 1-hexene, 1-octene or a mixture of them.

### ADVANTAGEOUS EFFECTS

If the oligomerization of ethylene is performed using the organochromium compound including the ligand compound and the catalyst composition of the present invention, a linear alpha-olefin may be produced with excellent productivity due to high catalyst activity, and high selectivity to 1-hexene and 1-octene.

### MODE FOR CARRYING OUT THE INVENTION

Hereinafter, the present invention will be described in more detail to assist the understanding of the present invention.

It will be understood that words or terms used in the present disclosure and claims shall not be interpreted as the meaning defined in commonly used dictionaries. It will be further understood that the words or terms should be interpreted as having a meaning that is consistent with their meaning in the context of the relevant art and the technical idea of the invention, based on the principle that an inventor may properly define the meaning of the words or terms to best explain the invention.

### Ligand Compound

The present invention provides a ligand compound which is applicable to a catalyst used for the oligomerization reaction of ethylene. If the ligand compound is applied to the oligomerization reaction of ethylene, particularly, to a catalyst composition for forming a linear alpha-olefin, excellent catalyst activity may be shown, while showing high selectivity to a linear alpha-olefin. Particularly, if compared to the conventional PNP-based catalyst, a solid polyethylene production amount is small under the same reaction conditions, and a linear alpha-olefin may be prepared more efficiently.

According to an embodiment of the present invention, the organochromium compound making a coordination bond with the ligand compound may be utilized for the preparation of a linear alpha-olefin using ethylene, and oligomerization reaction may be performed in the reaction under ethylene conditions to form a liquid type alpha-olefin, particularly, a liquid type 1-hexene or 1-octene with high selectivity. This is due to the increase of the selectivity to an alpha-olefin with a specific length through a transition state forming a metallacycle in the oligomerization reaction of ethylene.

According to an embodiment of the present invention, the ligand compound includes a diphosphino aminyl moiety, and aryl having a specific substituent is connected with the terminal of the diphosphino aminyl moiety, and accordingly, the ligand compound itself may have a type which may play the role of a strong electron donating group. Due to such structural features, the ligand compound may be applied to a catalyst system of oligomerizing ethylene to show high activity, particularly, high selectivity to 1-hexene, 1-octene, or the like. This is considered due to the interaction between adjacent chromium active sites. Particularly, if aryl substituted with a specific substituent is connected with the phosphor (P) atom of diphosphino aminyl, electron density may increase at the phosphor (P) atom and nitrogen (N) atom included in the diphosphino aminyl, and the electrical and three-dimensional properties of the whole ligand compound may change. Accordingly, there may be changes of the bond between the ligand and the chromium atom, the structure of the catalyst may be stabilized even further, an alpha-olefin may be formed with higher activity and selectivity by changing the energy of a transition state (activation energy) when compared to the conventional metallacycloheptane or metallacyclononane type, and the amount of by-products such as a solid alpha-olefin having a large molecular weight like a polyethylene wax (PE wax), may be reduced even further.

According to an embodiment of the present invention, the ligand compound is characterized in that phenyl positioned at the terminal of a diphosphino aminyl residue has a silyl group substituted with an alkyl group with a specific carbon number as a substituent, at a para position. The substituent substituted at the para position of the phenyl prevents the generation of an inactive species by the combination of two molecules of the ligand compound with the chromium atom, and reduces the rotatability of a nitrogen-phosphor bond to prevent the dissociation of the ligand compound in the catalyst, thereby producing a chromium catalyst having high stability and excellent activity and selectivity.

According to an embodiment of the present invention, in the ligand compound, to a nitrogen atom bonded to two phosphor atoms, a bulky substituent like a cycloalkyl group or a phenyl group is bonded, and the bulky substituent bonded to the nitrogen prevents the rotation of the bond between the nitrogen and the phosphor to improve the catalyst stability and activity even further.

According to an embodiment of the present invention, the ligand compound may particularly improve catalyst activity, selectivity and stability, because the phenyl positioned at the terminal of the diphosphino aminyl residue has a silyl group substituted with an alkyl group having a specific carbon number as a substituent at the para position, and because of the steric and electrical properties of a substituent substituted at a nitrogen atom. Particularly, in order to prepare 1-hexene and 1-octene, reaction through metallacycle is required, and metallacycloheptane and metallacyclononane intermediates are required to be formed effectively. In this case, if the metallacycle is not formed, but a general ethylene polymerization is performed, the selectivity may be markedly reduced, and there are problems of not performing a process operation by process problems due to the polymerization of polyethylene. In addition, though performing the reaction while forming metallacycle, the reaction may proceed to a direction forming by-products having 6 carbon atoms like methylcyclopentane or methylenecyclopentene. In addition, the preparation of complicated compounds having 10 to 14 carbon atoms by the participation of 1-hexene and 1-octene in the trimerization and tetramerization of ethylene again, may be the main forming reaction of the by-products, and to effectively reduce the phenomenon, it is important that the nitrogen atom of the ligand compound includes a suitable substituent. In relation, according to the ligand compound of the present invention, if the ligand compound introduces a substituent represented by R⁵ of Formula 1, particularly, a substituent introducing various substituents to a cycloalkyl group like a cyclohexyl group to the nitrogen atom, or introduces a ring compound including a heteroatom like oxygen and sulfur as a substituent, suitable steric hindrance may be formed, a polymerization reaction to polyethylene or the reaction by a mechanism proceeding to produce the by-products may be prevented, the reaction according to a metallacycle mechanism may be effectively induced, and high selectivity to 1-hexene and 1-octene may be secured. In addition, if the ligand compound includes the substituent on the nitrogen atom, due to steric effects and electrical effects, the activation energy of the reaction according to the metallacycle mechanism may be reduced, and thus, the reaction may be carried out more easily under the same temperature and the same pressure conditions, and the oligomerization reaction activity may be improved. Like this, the introduction of a novel substituent of the ligand compound according to the present invention enables the development of an oligomerization catalyst having markedly improved performance, markedly improves the level of commercialization and secures excellent economic feasibility.

According to an embodiment of the present invention, the ligand compound may be represented by Formula 1.

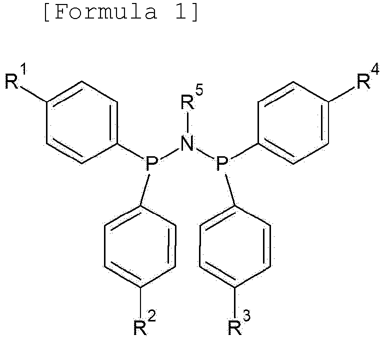

In Formula 1, R¹ to R⁴ may be each independently a trialkylsilyl group, where the alkyl group may be an alkyl group of 1 to 4 carbon atoms, and R⁵ may be a substituent represented by any one among Formulae 2 to 4.

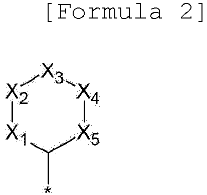

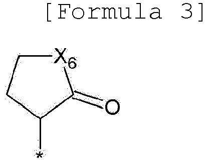

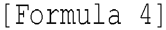

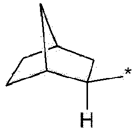

In Formulae 2 to 4, X₁ to X₆ may be each independently -(CR⁶R⁷)ₙ-, -O- or -S-, where all are not -O- or -S-simultaneously, and n may be an integer selected from 0 and 1 to 3, R⁶ and R⁷ may be each independently hydrogen, an alkyl group of 1 to 5 carbon atoms, a trifluoroalkyl group of 1 to 5 carbon atoms, an aryl group of 6 to 10 carbon atoms, or - NR⁸R⁹, R⁸ and R⁹ may be each independently hydrogen or an alkyl group of 1 to 5 carbon atoms, and * may be a part bonded to N of Formula 1.

In the present invention, "alkyl" means a hydrocarbon residue of a linear, cyclic or branched type, and particularly includes methyl, ethyl, n-propyl, isopropyl, n-butyl, isobutyl, t-butyl, n-pentyl, isopentyl and hexyl, according to the carbon number defined.

In the present invention, "trialkylsilyl" means a substituent represented by -SiR₃ where each R is independently alkyl, and the carbon number of the trialkylsilyl, if referred to, may mean the sum of all the carbon number of R.

In the present invention, "aryl" refers to an arbitrarily substituted benzene ring, or refers to a ring system which may be formed by fusing one or more arbitrary substituents, unless otherwise referred to. Examples of the arbitrary substituent include a substituted alkyl group of 1 to 2 carbon atoms, a substituted alkenyl group of 2 to 3 carbon atoms, a substituted alkynyl group of 2 to 3 carbon atoms, a heteroaryl group, a heterocyclic group, an aryl group, alkoxy having arbitrarily 1 to 3 fluorine substituents, aryloxy, aralkoxy, acyl, aroyl, heteroaroyl, acyloxy, aroyloxy, heteroaroyloxy, sulfanyl, sulfinyl, sulfonyl, aminosulfonyl, sulfonylamino, carboxyamide, aminocarbonyl, carboxy, oxo, hydroxyl, mercapto, amino, nitro, cyano, halogen or ureido. Such a ring or ring system may be arbitrarily fused with an aryl ring (for example, benzene ring), carbocyclic ring or heterocyclic ring, having arbitrarily one or more substituents. Non-limiting examples include phenyl, naphthyl, tetrahydronaphthyl, biphenyl, indanyl, anthracyl, phenanthryl and substituted derivatives thereof.

According to an embodiment of the present invention, R¹ to R⁴ may be each independently a tripropylsilyl group or a tributylsilyl group. That is, in the ligand compound, a phenyl group positioned at the terminal of a diphosphino aminyl residue may have a silyl group substituted with an alkyl group of 3 or 4 carbon atoms, at a para position, as a substituent.

According to an embodiment of the present invention, R⁵ may be a substituent represented by any one among Formulae 2 to 4 above, X₁, X₂ and X₅ may be each independently - (CR⁶R⁷)ₙ-, X₃ and X₄ may be each independently -(CR⁶R⁷)ₙ- or -O-, where X₃ and X₄ may not be -O- simultaneously, X₆ may be -S-, R⁶ and R⁷ may be each independently hydrogen, a trifluoroalkyl group of 1 to 5 carbon atoms, an aryl group of 6 to 10 carbon atoms, or -NR⁸R⁹, and R⁸ and R⁹ may be each independently hydrogen or an alkyl group of 1 to 5 carbon atoms. In addition, n may be 1 or 2, and all may not be 2 simultaneously.

According to an embodiment of the present invention, in the ligand compound, R⁵ may be a substituent represented by Formula 2 above, X₁ and X₅ may be -CH₂-, X₂ to X₄ may be each independently -(CR⁶R⁷)ₙ-, -O- or -S-, where all may not be -O- or -S- simultaneously, R⁶ and R⁷ may be each independently hydrogen, a trifluoroalkyl group of 1 to 5 carbon atoms, an aryl group of 6 to 10 carbon atoms, or -NR⁸R⁹, and R⁸ and R⁹ may be each independently hydrogen or an alkyl group of 1 to 3 carbon atoms. In a particular embodiment, the ligand compound represented by Formula 1 may be one selected from the group consisting of the ligand compounds represented by Formula 2-1 to Formula 2-14.

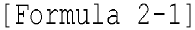

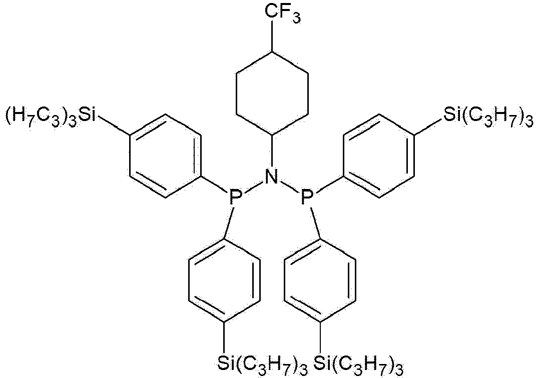

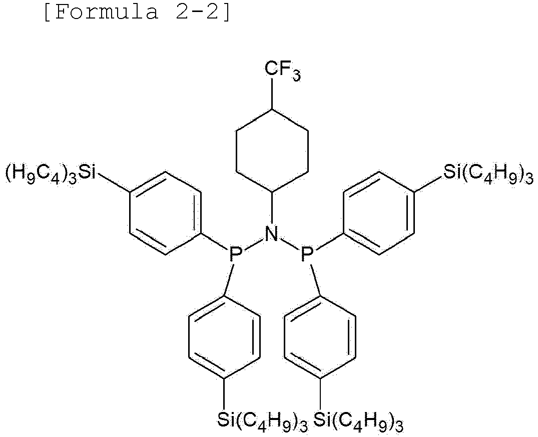

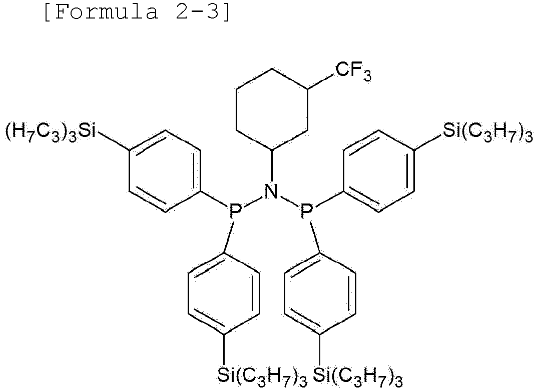

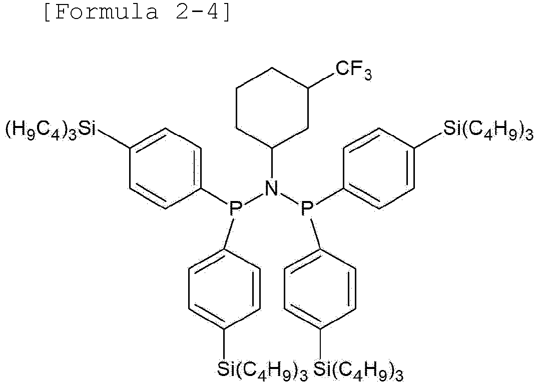

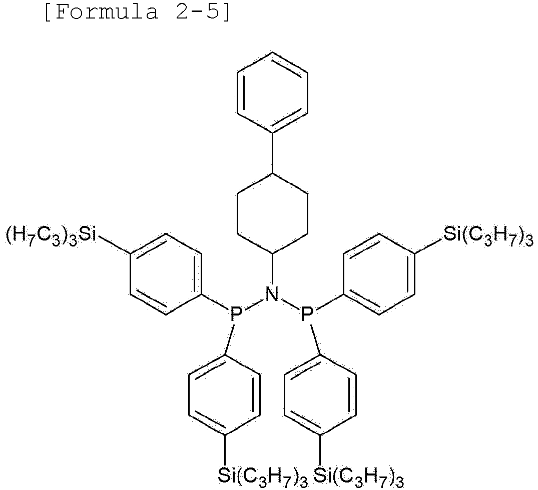

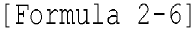

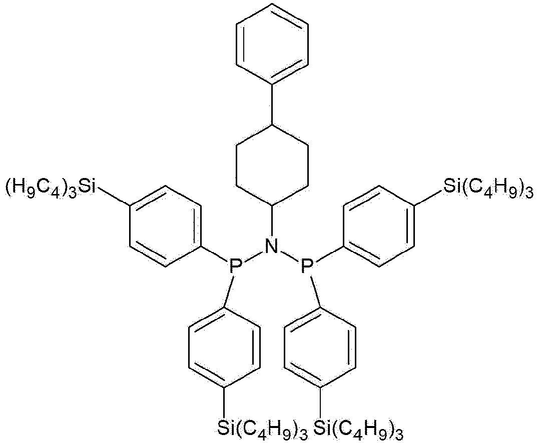

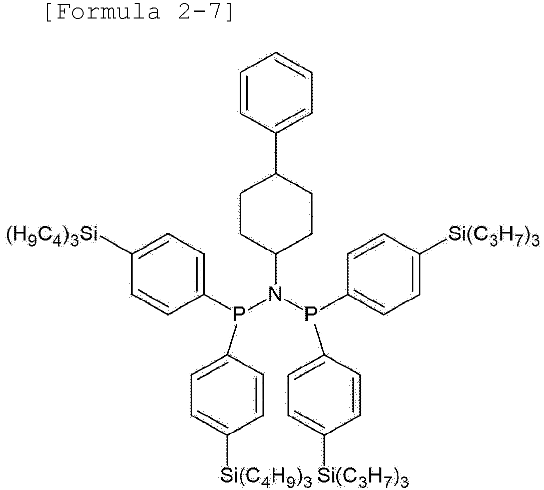

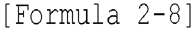

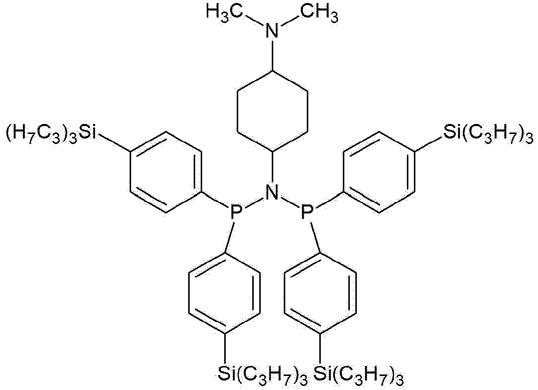

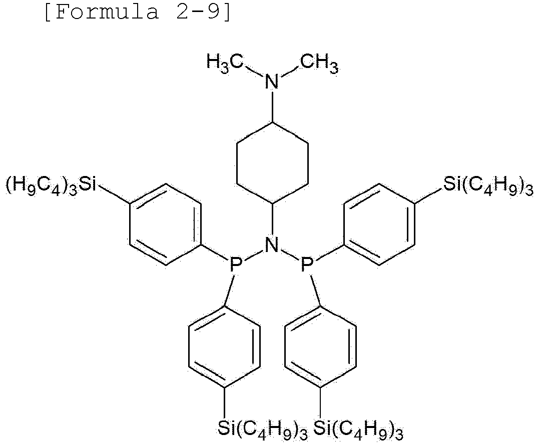

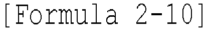

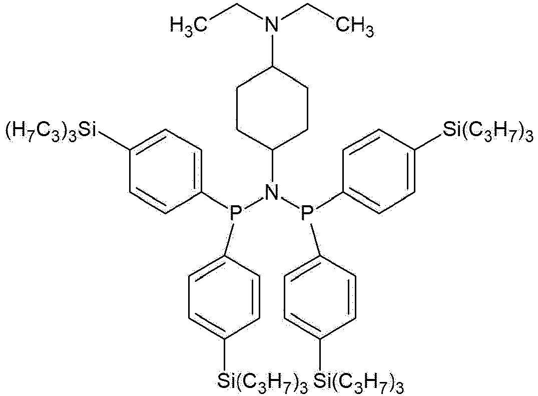

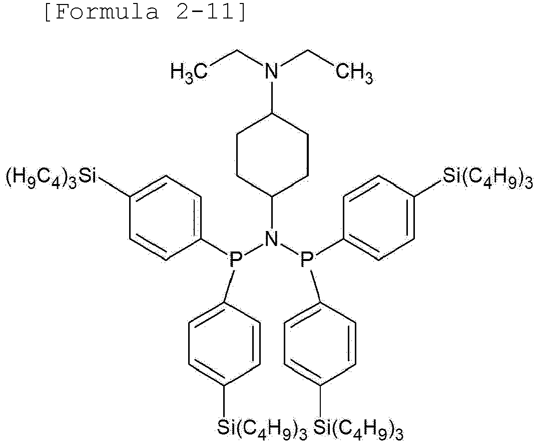

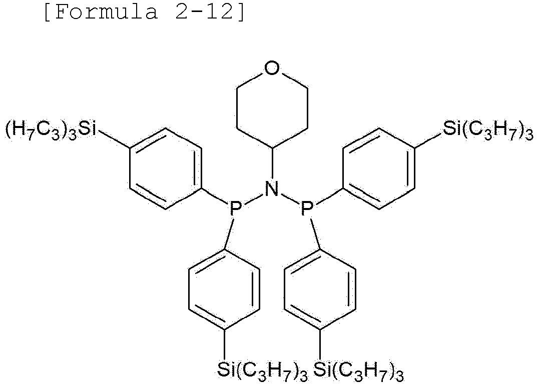

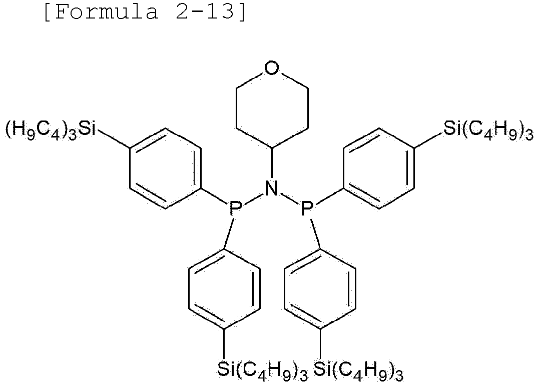

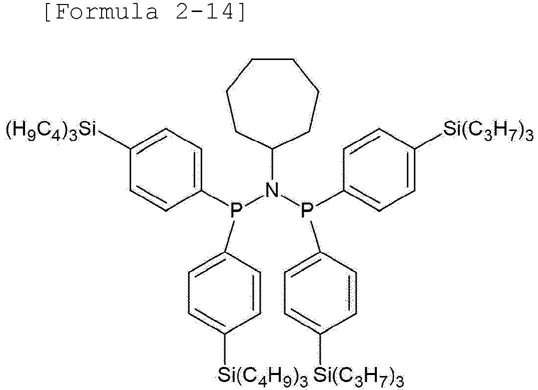

According to an embodiment of the present invention, in the ligand compound, R⁵ may be a substituent represented by Formula 3, and X₆ may be -O- or -S-. In a particular embodiment, the ligand compound represented by Formula 1 may be represented by Formula 3-1 or Formula 3-2.

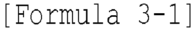

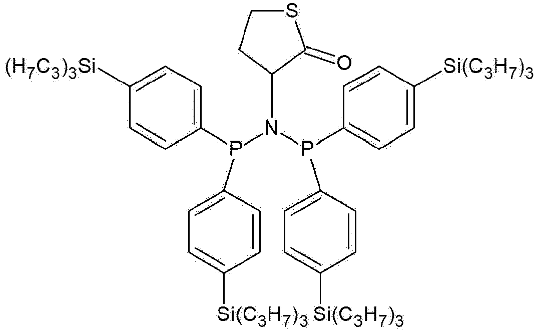

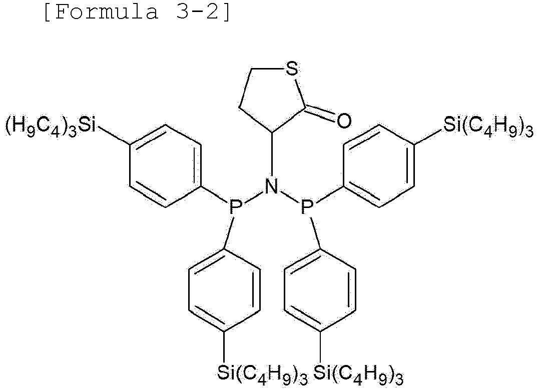

According to an embodiment of the present invention, the ligand compound may be represented by Formula 4-1 or Formula 4-2.

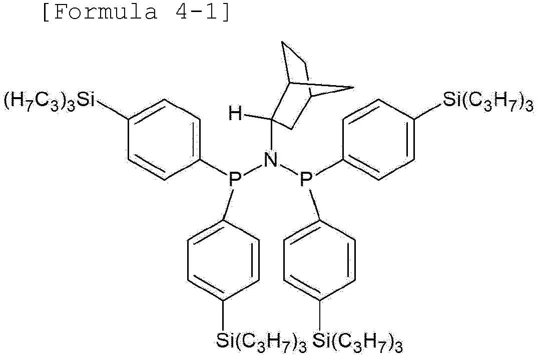

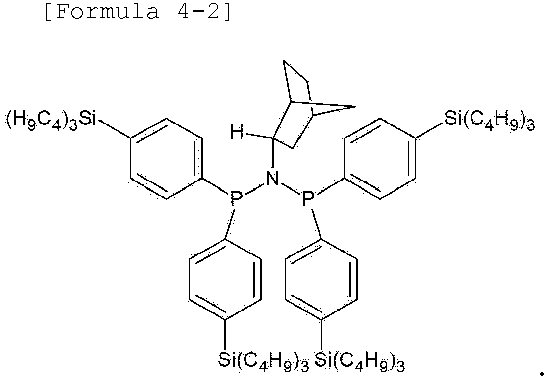

According to an embodiment of the present invention, the ligand compound may be accomplished by various combinations within a range satisfying the above-described conditions in addition to the above embodiments, and any compounds may be applicable as the ligand compound of the present invention as long as it is represented by Formula 1.

### Organochromium compound and catalyst composition

The present invention provides an organochromium compound including the ligand compound represented by Formula 1 and chromium (Cr) making a coordination bond with the ligand compound.

According to an embodiment of the present invention, the organochromium compound is the chromium complex compound of the ligand compound and may have a type where the chromium of a chromium source makes coordination bonds with unshared electron pairs of one or more among N and two P in the ligand compound represented by Formula 1. That is, in the structure, the phosphor atom or nitrogen atom of a diphosphino aminyl residue provide the chromium atom with an unshared electron pair, and particularly, a bidentated state in which two unshared electron pairs are shared, may be preferable. Such an organochromium compound may be applied to a catalyst system for the oligomerization reaction of ethylene to show excellent catalyst activity and high selectivity to 1-hexene and 1-octene.

In the present invention, a "catalyst composition" means a state obtainable as a catalyst composition having activity by adding three components including a chromium source, a ligand compound and a cocatalyst, or two components of a transition metal compound and a cocatalyst in an arbitrary order. Here, the catalyst composition may be referred to as a catalyst system, and in the present invention, the catalyst composition and the catalyst system show the same meaning. The three components or two components of the catalyst composition may be added in the presence or absence of a solvent and a monomer, and may be used in a supported or unsupported state.

The present invention provides a catalyst compound including the ligand compound, chromium and a cocatalyst. The ligand compound represented by Formula 1 and chromium may be coordinated as described above to form an organochromium compound. That is, the catalyst system may be a three-component catalyst system including chromium, the ligand compound represented by Formula 1 and a cocatalyst, or a two-component catalyst system including the organochromium compound and a cocatalyst. In a particular embodiment, the catalyst composition may include an organochromium compound including the ligand compound, chromium making a coordination bond with the ligand compound, and a cocatalyst. In addition, the catalyst composition may include a chromium compound in which a partial component of the cocatalyst is bonded to the organochromium compound.

According to an embodiment of the present invention, the chromium may be derived from a chromium source, and the chromium source may be an organic or inorganic chromium compound with an oxidation state of 0 to 6. In a particular embodiment, the chromium source may be a chromium metal, or a compound in which an arbitrary organic or inorganic radical is bonded to chromium. Here, the organic radical may be alkyl, alkoxy, ester, ketone, amido, carboxylate radical, or the like, having 1 to 20 carbon atoms per radical, and the inorganic radical may be halide, sulfate, oxide, or the like.

According to an embodiment of the present invention, the chromium source is a compound showing high activity for the oligomerization of an olefin and easily used and obtained, and may be one or more compounds selected from the group consisting of chromium(III) acetylacetonate, chromium(III) chloride tetrahydrofuran, chromium(III) 2-ethylhexanoate, chromium(III) acetate, chromium(III) butyrate, chromium(III) pentanoate, chromium(III) laurate, chromium(III) tris(2,2,6,6-tetramethyl-3,5-heptanedionate) and chromium(III) stearate.

According to an embodiment of the present invention, the cocatalyst may be one or more selected from the group consisting of the compounds represented by Formula 5 to Formula 8.

[Formula 5] -[Al(R¹⁰)-O]ₐ-

In Formula 5, each R¹⁰ is independently a halogen group, a hydrocarbyl group of 1 to 20 carbon atoms, or a hydrocarbyl group of 1 to 20 carbon atoms, which is substituted with a halogen group, and a is an integer of 2 or more.

[Formula 6] E(R¹¹)₃

In Formula 6, E is aluminum or boron, and each R¹¹ is independently hydrogen, a halogen group, a hydrocarbyl group of 1 to 20 carbon atoms, or a hydrocarbyl radical of 1 to 20 carbon atoms, which is substituted with a halogen group.

[Formula 7] [L-H]⁺[G(Y)_{4]}⁻

[Formula 8] [L]⁺[G(Y)₄]⁻

In Formula 7 and Formula 8, L is a neutral or cationic Lewis acid, [L-H]⁺ is a bronsted acid, G is an element in group 13, and each Y is independently a substituted or unsubstituted alkyl group of 1 to 20 carbon atoms or a substituted or unsubstituted aryl group of 6 to 20 carbon atoms, where if the alkyl group or the aryl group is substituted, the substituent is a halogen group, a hydrocarbyl group of 1 to 20 carbon atoms, an alkoxy group of 1 to 20 carbon atoms, or an aryloxy group of 6 to 20 carbon atoms.

According to an embodiment of the present invention, the catalyst composition may be prepared by numbers of methods. In a particular embodiment, first, the catalyst composition may be prepared by including a step of contacting the organochromium compound with the compound represented by Formula 5 or Formula 6. Second, the catalyst composition may be prepared by including a step of contacting the organochromium compound with the compound represented by Formula 5 or Formula 6 to obtain a mixture; and a step of adding the compound represented by Formula 7 or Formula 8 to the mixture. Third, the catalyst composition may be prepared by including a step of contacting the organochromium compound with the compound represented by Formula 7 or Formula 8. Fourth, the catalyst composition may be prepared by including a step of contacting the organochromium compound with the compound represented by Formula 7 or Formula 8 to obtain a mixture; and a step of adding the compound represented by Formula 5 or Formula 6 to the mixture. Fifth, the catalyst composition may be prepared by including a step of contacting the chromium source with the compound represented by Formula 7 or Formula 8 to obtain a reaction product, and a step of contacting the reaction product with the ligand compound.

According to an embodiment of the present invention, in the first method or the third method among the preparation methods of the catalyst composition, the molar ratio of the compound represented by Formula 5 or Formula 6 in contrast to the organochromium compound may be 1: 2 to 5,000, particularly, 1: 100 to 3,000, more particularly, 1: 300 to 1,500, and within this range, the alkylation of the organochromium compound may be completely performed, the activity of the catalyst composition may be improved, the degradation of the activation of the alkylated organochromium compound due to the side reactions between remaining alkylating agents, may be prevented, and at the same time, the economic feasibility and the purity of a linear alpha-olefin prepared may be improved.

According to an embodiment of the present invention, in the case of the second method among the preparation methods of the catalyst composition, the molar ratio of the compound represented by Formula 7 or Formula 8 in contrast to the organochromium compound may be 1: 1 to 500, particularly, 1: 1 to 50, more particularly, 1: 1 to 1: 25, and within this range, the amount of an activating agent is sufficient, the activation of a metal compound may be completely performed, and the activity of the catalyst composition may be improved, the activating agent remained may be minimized, and the purity of a linear alpha-olefin prepared may be improved.

According to an embodiment of the present invention, the compound represented by Formula 5 may be alkylaluminoxane, and particular examples may include methylaluminoxane, ethylaluminoxane, isobutylaluminoxane, butylaluminoxane, or the like, more particularly, methylaluminoxane.

According to an embodiment of the present invention, the compound represented by Formula 6 may be trialkyl aluminum, dialkyl aluminum halide, alkyl aluminum dihalide, dialkyl aluminum hydride, alkyl aluminum dihydride, trialkylboron, or the like. In a particular embodiment, the compound represented by Formula 6 may be trialkyl aluminum such as trimethylaluminum, triethylaluminum, triisobutylaluminum, tripropylaluminum, tributylaluminum, triisopropylaluminum, tri-s-butylaluminum, tricyclopentylaluminum, tripentylaluminum, triisopentylaluminum, trihexylaluminum, trioctylaluminum, ethyldimethylaluminum, methyldiethylaluminum, triphenylaluminum, and tri-p-tolylaluminum; dialkylaluminum halide such as diethylaluminum chloride; dialkyl aluminum hydride such as diethyl aluminum hydride, di-n-propyl aluminum hydride, diisopropyl aluminum hydride, di-n-butyl aluminum hydride, dibutyl aluminum hydride, diisobutyl aluminum hydride (DIBAH), di-n-octyl aluminum hydride, diphenyl aluminum hydride, di-p-tolyl aluminum hydride, dibenzyl aluminum hydride, phenylethyl aluminum hydride, phenyl-n-propyl aluminum hydride, phenylisopropyl aluminum hydride, phenyl-n-butyl aluminum hydride, phenylisobutyl aluminum hydride, phenyl-n-octyl aluminum hydride, p-tolylethyl aluminum hydride, p-tolyl-n-propyl aluminum hydride, p-tolylisopropyl aluminum hydride, p-tolyl-n-butyl aluminum hydride, p-tolylisobutyl aluminum hydride, p-tolyl-n-octyl aluminum hydride, benzylethyl aluminum hydride, benzyl-n-propyl aluminum hydride, benzylisopropyl aluminum hydride, benzyl-n-butyl aluminum hydride, benzylisobutyl aluminum hydride and benzyl-n-octyl aluminum hydride; alkyl aluminum dihydride such as n-propyl aluminum dihydride, isopropyl aluminum dihydride, n-butyl aluminum dihydride, isobutyl aluminum dihydride, and n-octylaluminum dihydride; and trialkylboron such as trimethylboron, triethylboron, triisobutylboron, tripropylboron and tributylboron.

According to an embodiment of the present invention, the compound represented by Formula 7 or Formula 8 may be trimethylammonium tetraphenylborate, triethylammonium tetraphenylborate, tripropylammonium tetraphenylborate, tributylammonium tetraphenylborate, N,N-dimethylanilinium tetraphenylborate, N,N-diethylanilinium tetraphenylborate, trimethylammonium tetra(p-tolyl)borate, triethylammonium tetra(p-tolyl)borate, tripropylammonium tetra(p-tolyl)borate, tributylammonium tetra(p-tolyl)borate, N,N-dimethylanilinium tetra(p-tolyl)borate, N,N-diethylanilinium tetra(p-tolyl)borate, trimethylammonium tetra(o,p-dimethylphenyl)borate, triethylammonium tetra(o,p-dimethylphenyl)borate, tripropylammonium tetra(o,p-dimethylphenyl)borate, tributylammonium tetra(o,p-dimethylphenyl)borate, N,N-dimethylanilinium tetra(o,p-dimethylphenyl)borate, N,N-diethylailinium tetra(o,p-dimethylphenyl)borate, trimethylammonium tetrakis(p-trifluoromethylphenyl)borate, triethylammonium tetrakis(p-trifluoromethylphenyl)borate, tripropylammonium tetrakis(p-trifluoromethylphenyl)borate, tributylammonium tetrakis(p-trifluoromethylphenyl)borate, N,N-dimethylanilinium tetrakis(p-trifluoromethylphenyl)borate, N,N-diethylailinium tetrakis(p-trifluoromethylphenyl)borate, trimethylammonium tetrakis(pentafluorophenyl)borate, triethylammonium tetrakis(pentafluorophenyl)borate, tripropylammonium tetrakis(pentafluorophenyl)borate, tributylammonium tetrakis(pentafluorophenyl)borate, N,N-dimethylanilinium tetrakis(pentafluorophenyl)borate, N,N-diethylanilinium tetrais(pentafluorophenyl)borate, N,N-dioctadecylanilinium tetrakis(pentafluorophenyl)borate, trimethylphosphonium tetraphenylborate, triethylphosphonium tetraphenylborate, tripropylphosphonium tetraphenylborate, tributylphosphonium tetraphenylborate, trimethylcarbonium tetraphenylborate, triethylcarbonium tetraphenylborate, tripropylcarbonium tetraphenylborate, tributylcarbonium tetraphenylborate, trimethylammonium tetraphenylaluminate, triethylammonium tetraphenylaluminate, tripropylammonium tetraphenylaluminate, tributylammonium tetraphenylaluminate, trimethylammonium tetra(p-tolyl)aluminate, triethylammonium tetra(p-tolyl)aluminate, tripropylammonium tetra(p-tolyl)aluminate, tributylammonium tetra(p-tolyl)aluminate, or the like.

According to an embodiment of the present invention, the amount ratio of the components constituting the catalyst composition may be determined considering the catalyst activity and the selectivity to a linear alpha-olefin. In a particular embodiment, if the catalyst composition is a three-component catalyst composition, the molar ratio of diphosphino aminyl residue of the ligand compound: chromium source: cocatalyst may be controlled to about 1: 1: 1 to about 10: 1: 10,000, or about 1: 1: 100 to 5: 1: 3,000. In addition, if the catalyst composition is a two-component catalyst composition, the molar ratio of diphosphino aminyl residue of the organochromium compound: cocatalyst may be controlled to 1: 1 to 1: 10,000, or 1: 1 to 1: 5,000 or 1: 1 to 1: 3,000.

According to an embodiment of the present invention, the reaction solvent during preparing the catalyst composition may use a hydrocarbon-based solvent such as pentane, hexane and heptane; and an aromatic solvent such as benzene and toluene.

According to an embodiment of the present invention, the components constituting the catalyst composition may be added simultaneously or in an arbitrary order in the presence or absence of a suitable solvent and monomer, and may act as a catalyst composition having activity. In this case, the suitable solvent may include heptane, toluene, cyclohexane, methylcyclohexane, 1-hexene, 1-octene, diethyl ether, tetrahydrofuran, acetonitrile, dichloromethane, chloroform, chlorobenzene, methanol, acetone, or the like.

According to an embodiment of the present invention, the organochromium compound and the cocatalyst may be used in supported types in supports, and in this case, the support may be silica or alumina.

According to an embodiment of the present invention, the catalyst composition may further include a support. In a particular embodiment, the ligand compound represented by Formula 1 may be applied for the oligomerization of ethylene, in a supported type in a support. The support may be a metal, a metal salt or a metal oxide, applied in a supported catalyst, and in a particular embodiment, the support may be silica, silica-alumina, silica-magnesia, or the like, and may include the oxide, carbonate, sulfate, or nitrate component of a metal such as Na₂O, K₂CO₃, BaSO₄, and Mg(NO₃)₂.

According to an embodiment of the present invention, the catalyst composition may be used for the trimerization or tetramerization reaction of ethylene, and may produce 1-hexene or 1-octene with high selectivity by the above-description.

### Method for oligomerizing ethylene

The present invention provides a method for preparing a linear alpha-olefin as a method for oligomerizing ethylene, comprising a step of oligomerizing ethylene in the presence of the catalyst composition (S10).

In the present invention, "oligomerization" means oligomerizing olefins. According to the number of olefins polymerized, the oligomerization is referred to as trimerization or tetramerization, and is collectively referred to as multimerization. Particularly, in the present disclosure, the oligomerization may mean selective preparation of 1-hexene and 1-octene, which are main comonomers of a LLDPE from ethylene.

According to an embodiment of the present invention, the oligomerization reaction of ethylene may be trimerization or tetramerization of ethylene, and 1-hexene or 1-octene may be formed as the resultant reaction product, and accordingly, the linear alpha-olefin may be 1-hexene, 1-octene or a mixture thereof.

According to an embodiment of the present invention, the method of oligomerizing ethylene may be performed by using ethylene as a raw material and applying the above-described catalyst composition, a common apparatus and contacting technique. In a particular embodiment, the oligomerization reaction of ethylene may include homogeneous liquid phase reaction in the presence or absence of an inert solvent, slurry reaction by which a portion or whole of the catalyst composition is not dissolved, bulk phase reaction in which an alpha-olefin product acts as a main medium, or gas phase reaction.

According to an embodiment of the present invention, the oligomerization reaction of ethylene may be performed under an inert solvent. Particular examples of the inert solvent may include benzene, toluene, xylene, cumene, chlorobenzene, dichlorobenzene, heptane, cyclohexane, methylcyclohexane, methylcyclopentane, n-hexane, 1-hexene, 1-octene, 2,2,4-trimethylpentane, or the like.

According to an embodiment of the present invention, the oligomerization reaction of ethylene may be performed under a temperature of 0°C to 200°C, or 0°C to 150°C, or 30°C to 100°C, or 50°C to 100°C. In addition, the reaction may be performed under a pressure of 15 psig to 3000 psig, or 15 psig to 1500 psig, or 15 psig to 1000 psig.

Hereinafter, embodiments of the present invention will be explained in more detail so that a person skilled in the art to which the present invention belongs, may easily perform. However, the embodiments may be accomplished by various different types and is not limited to the embodiments explained herein.

### Synthetic Examples and Comparative Synthetic Examples

### Synthetic Example 1: Synthesis of ligand compound represented by Formula 2-1

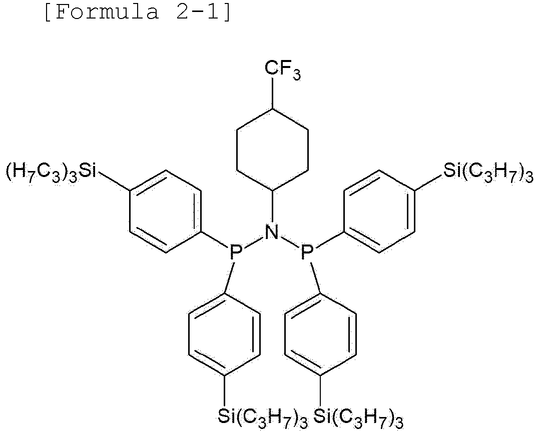

20 mmol (2 eq) of 4-(tripropylsilyl)phenyl bromide was dissolved in 20 ml of tetrahydrofuran and cooled to -78°C. While maintaining the temperature, 20 mmol (2 eq) of n-butyllithium was added thereto dropwisely, followed by stirring for 3 hours. Then, 10 mmol (1 eq) of dichloro(diethylamino)phosphine dissolved in 10 ml of tetrahydrofuran was added thereto dropwisely, and the temperature was raised to room temperature, followed by stirring overnight. Then, the solvent was removed using vacuum, and an intermediate obtained without additional purification was dissolved in 30 ml of hexane, and HCl (in ether, 2 eq) was added thereto. The resultant was subjected to stirring for 15 minutes and filtering. 2.1 mmol (2.1 eq) of an intermediate obtained by drying the filtrate in vacuum was dissolved in 3.8 ml of dichloromethane, and 3 mmol (3 eq) of triethylamine was added thereto. Then, 1 mmol (1 eq) of 4-(trifluoromethyl)cyclohexyl amine dissolved in 3.8 ml of dichloromethane was slowly added to a reaction system, followed by stirring overnight at room temperature. Then, the solvent was removed using vacuum, and the resultant was dissolved in 7.6 ml of hexane and loaded on the top of silica. Silica filtration was performed using hexane with 1 vol% of triethylamine, and the solution thus obtained was concentrated to obtain the ligand compound represented by Formula 2-1.

### <Ligand compound represented by Formula 2-1>

N-(bis(4-(tripropylsilyl)phenyl)phosphaneyl)-N-(4-(trifluoromethyl)cyclohexyl)-1,1-bis(4-(tripropylsilyl)phenyl)phosphanamine)
¹H NMR (500 MHz, C₆D₆): δ 8.00-7.20 (m, 16H), 3.31(pent, 1H), 2.52-2.38(m, 2H), 2.00-1.52(m, 4H), 1.40-1.25(m, 27H), 0.98(t, 36H), 0.79-0.73(m, 24H)

### Synthetic Example 2: Synthesis of ligand compound represented by Formula 2-3

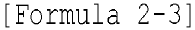

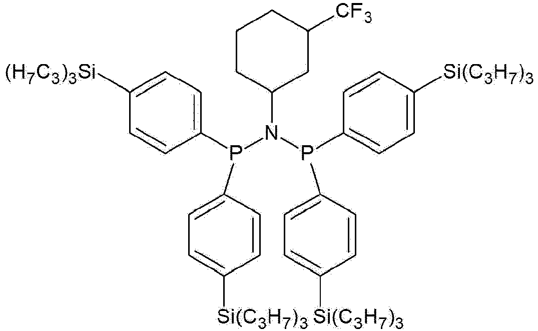

20 mmol (2 eq) of 4-(tripropylsilyl)phenyl bromide was dissolved in 20 ml of tetrahydrofuran and cooled to -78°C. While maintaining the temperature, 20 mmol (2 eq) of n-butyllithium was added thereto dropwisely, followed by stirring for 3 hours. Then, 10 mmol (1 eq) of dichloro(diethylamino)phosphine dissolved in 10 ml of tetrahydrofuran was added thereto dropwisely, and the temperature was raised to room temperature, followed by stirring overnight. Then, the solvent was removed using vacuum, and an intermediate obtained without additional purification was dissolved in 30 ml of hexane, and HCl (in ether, 2 eq) was added thereto. The resultant was subjected to stirring for 15 minutes and filtering. 2.1 mmol (2.1 eq) of an intermediate obtained by drying the filtrate in vacuum was dissolved in 3.8 ml of dichloromethane, and 3 mmol (3 eq) of triethylamine was added thereto. Then, 1 mmol (1 eq) of 3-(trifluoromethyl)cyclohexyl amine dissolved in 3.8 ml of dichloromethane was slowly added to a reaction system, followed by stirring overnight at room temperature. Then, the solvent was removed using vacuum, and the resultant was dissolved in 7.6 ml of hexane and loaded on the top of silica. Silica filtration was performed using hexane with 1 vol% of triethylamine, and the solution thus obtained was concentrated to obtain the ligand compound represented by Formula 2-3.

### <Ligand compound represented by Formula 2-3>

N-(bis(4-(tripropylsilyl)phenyl)phosphaneyl)-N-(3-(trifluoromethyl)cyclohexyl)-1,1-bis(4-(tripropylsilyl)phenyl)phosphanamine)
¹H NMR (500 MHz, C₆D₆) : δ 8.00-7.20(m, 16H), 3.95-3.82(m, 0.18H), 3.33(pent, 0.82H), 2.20-2.00(m, 2H), 1.70-1.55(m, 2H), 1.53-1.25(m, 27H), 1.05-0.92(m, 36H), 0.80-0.70(m, 24H)

### Synthetic Example 3: Synthesis of ligand compound represented by Formula 2-5

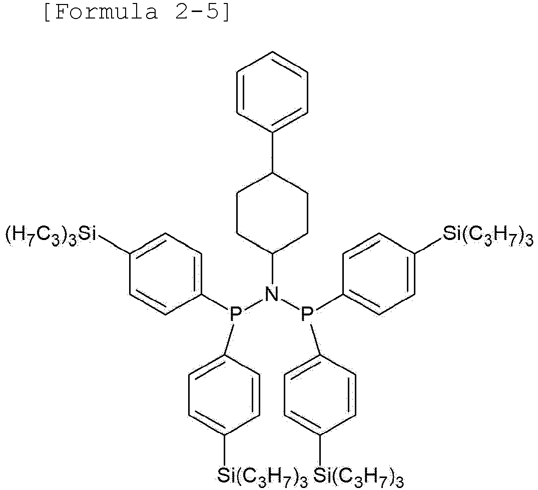

20 mmol (2 eq) of 4-(tripropylsilyl)phenyl bromide was dissolved in 20 ml of tetrahydrofuran and cooled to -78°C. While maintaining the temperature, 20 mmol (2 eq) of n-butyllithium was added thereto dropwisely, followed by stirring for 3 hours. Then, 10 mmol (1 eq) of dichloro(diethylamino)phosphine dissolved in 10 ml of tetrahydrofuran was added thereto dropwisely, and the temperature was raised to room temperature, followed by stirring overnight. Then, the solvent was removed using vacuum, and an intermediate obtained without additional purification was dissolved in 30 ml of hexane, and HCl (in ether, 2 eq) was added thereto. The resultant was subjected to stirring for 15 minutes and filtering. 2.1 mmol (2.1 eq) of an intermediate obtained by drying the filtrate in vacuum was dissolved in 3.8 ml of dichloromethane, and 3 mmol (3 eq) of triethylamine was added thereto. Then, 1 mmol (1 eq) of 4-phenylcyclohexylamine dissolved in 3.8 ml of dichloromethane was slowly added to a reaction system, followed by stirring overnight at room temperature. Then, the solvent was removed using vacuum, and the resultant was dissolved in 7.6 ml of hexane and loaded on the top of silica. Silica filtration was performed using hexane with 1 vol% of triethylamine, and the solution thus obtained was concentrated to obtain the ligand compound represented by Formula 2-5.

### <Ligand compound represented by Formula 2-5>

N-(bis(4-(tripropylsilyl)phenyl)phosphaneyl)-N-(4-phenylcyclohexyl)-1,1-bis(4-(tripropylsilyl)phenyl)phosphanamine)
¹H NMR (500 MHz, C₆D₆) : δ 8.00-6.90(m, 21H), 3.65-3.45(m, 0.88H), 3.40-3.28(m, 0.12H), 2.80-2.25(m, 4H), 2.10-1.47(m, 5H), 1.45-1.27(m, 24H), 0.99-0.92(m, 36H), 0.79-0.72(m, 24H)

### Synthetic Example 4: Synthesis of ligand compound represented by Formula 2-8

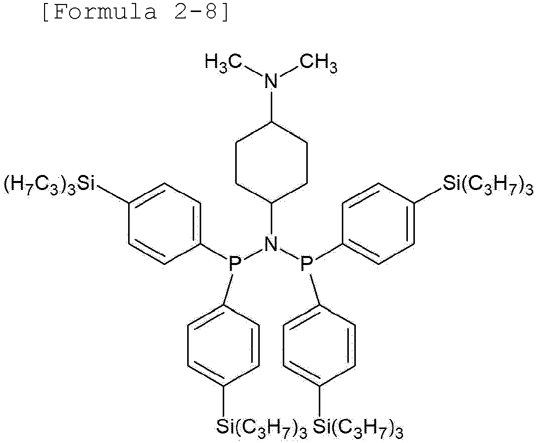

20 mmol (2 eq) of 4-(tripropylsilyl)phenyl bromide was dissolved in 20 ml of tetrahydrofuran and cooled to -78°C. While maintaining the temperature, 20 mmol (2 eq) of n-butyllithium was added thereto dropwisely, followed by stirring for 3 hours. Then, 10 mmol (1 eq) of dichloro(diethylamino)phosphine dissolved in 10 ml of tetrahydrofuran was added thereto dropwisely, and the temperature was raised to room temperature, followed by stirring overnight. Then, the solvent was removed using vacuum, and an intermediate obtained without additional purification was dissolved in 30 ml of hexane, and HCl (in ether, 2 eq) was added thereto. The resultant was subjected to stirring for 15 minutes and filtering. 2.1 mmol (2.1 eq) of an intermediate obtained by drying the filtrate in vacuum was dissolved in 3.8 ml of dichloromethane, and 3 mmol (3 eq) of triethylamine was added thereto. Then, 1 mmol (1 eq) of 4-(dimethylamino)cyclohexyl amine dissolved in 3.8 ml of dichloromethane was slowly added to a reaction system, followed by stirring overnight at room temperature. Then, the solvent was removed using vacuum, and the resultant was dissolved in 7.6 ml of hexane and loaded on the top of silica. Silica filtration was performed using hexane with 1 vol% of triethylamine, and the solution thus obtained was concentrated to obtain the ligand compound represented by Formula 2-8.

### <Ligand compound represented by Formula 2-8>

N¹,N¹-bis(bis(4-(tripropylsilyl)phenyl)phosphaneyl)-N⁴,N⁴-dimethylcyclohexane-1,4-diamine
¹H NMR (500 MHz, C₆D₆) : δ 8.00-7.18(m, 16H), 3.25 (pent, 1H), 2.55-2.42(m, 2H), 2.34-2.23(m, 1H), 2.12(s, 6H), 1.85-1.65(m, 4H), 1.40-1.25(m, 24H), 0.98(t, 36H), 0.87-0.75(m, 24H)

### Synthetic Example 5: Synthesis of ligand compound represented by Formula 2-10

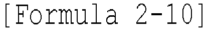

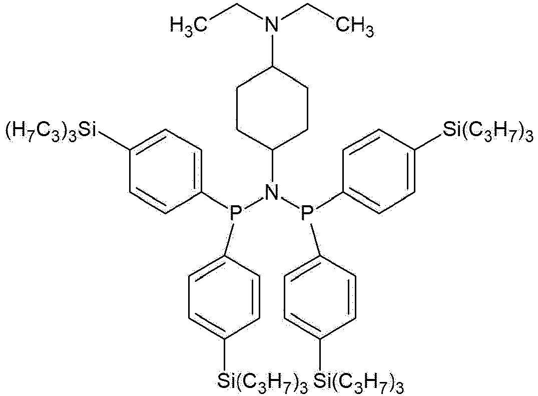

20 mmol (2 eq) of 4-(tripropylsilyl)phenyl bromide was dissolved in 20 ml of tetrahydrofuran and cooled to -78°C. While maintaining the temperature, 20 mmol (2 eq) of n-butyllithium was added thereto dropwisely, followed by stirring for 3 hours. Then, 10 mmol (1 eq) of dichloro(diethylamino)phosphine dissolved in 10 ml of tetrahydrofuran was added thereto dropwisely, and the temperature was raised to room temperature, followed by stirring overnight. Then, the solvent was removed using vacuum, and an intermediate obtained without additional purification was dissolved in 30 ml of hexane, and HCl (in ether, 2 eq) was added thereto. The resultant was subjected to stirring for 15 minutes and filtering. 2.1 mmol (2.1 eq) of an intermediate obtained by drying the filtrate in vacuum was dissolved in 3.8 ml of dichloromethane, and 3 mmol (3 eq) of triethylamine was added thereto. Then, 1 mmol (1 eq) of 4-(diethylamino)cyclohexyl amine dissolved in 3.8 ml of dichloromethane was slowly added to a reaction system, followed by stirring overnight at room temperature. Then, the solvent was removed using vacuum, and the resultant was dissolved in 7.6 ml of hexane and loaded on the top of silica. Silica filtration was performed using hexane with 1 vol% of triethylamine, and the solution thus obtained was concentrated to obtain the ligand compound represented by Formula 2-10.

### <Ligand compound represented by Formula 2-10>

N¹,N¹-bis(bis(4-(tripropylsilyl)phenyl)phosphaneyl)-N⁴,N⁴-diethylcyclohexane-1,4-diamine
¹H NMR (500 MHz, C₆D₆): δ 8.00-7.18 (m, 16H), 3.21 (pent, 1H), 2.54-2.40(m, 2H), 2.30-2.20(m, 1H), 2.18(q, 4H), 1.83-1.60(m, 4H), 1.40-1.23(m, 30H), 0.95(t, 36H), 0.85-0.74(m, 24H)

### Synthetic Example 6: Synthesis of ligand compound represented by Formula 2-12

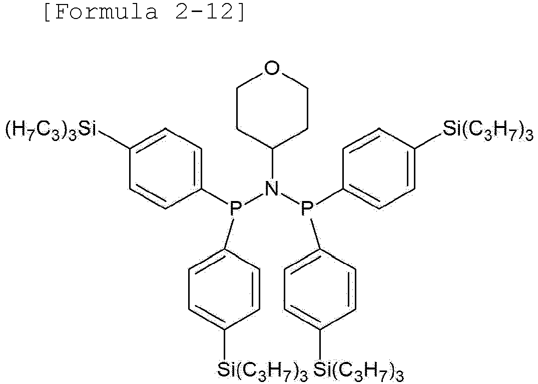

20 mmol (2 eq) of 4-(tripropylsilyl)phenyl bromide was dissolved in 20 ml of tetrahydrofuran and cooled to -78°C. While maintaining the temperature, 20 mmol (2 eq) of n-butyllithium was added thereto dropwisely, followed by stirring for 3 hours. Then, 10 mmol (1 eq) of dichloro(diethylamino)phosphine dissolved in 10 ml of tetrahydrofuran was added thereto dropwisely, and the temperature was raised to room temperature, followed by stirring overnight. Then, the solvent was removed using vacuum, and an intermediate obtained without additional purification was dissolved in 30 ml of hexane, and HCl (in ether, 2 eq) was added thereto. The resultant was subjected to stirring for 15 minutes and filtering. 2.1 mmol (2.1 eq) of an intermediate obtained by drying the filtrate in vacuum was dissolved in 3.8 ml of dichloromethane, and 3 mmol (3 eq) of triethylamine was added thereto. Then, 1 mmol (1 eq) of tetrahydro-2H-pyran-4-yl amine dissolved in 3.8 ml of dichloromethane was slowly added to a reaction system, followed by stirring overnight at room temperature. Then, the solvent was removed using vacuum, and the resultant was dissolved in 7.6 ml of hexane and loaded on the top of silica. Silica filtration was performed using hexane with 1 vol% of triethylamine, and the solution thus obtained was concentrated to obtain the ligand compound represented by Formula 2-12.

### <Ligand compound represented by Formula 2-12>

N-(bis(4-(tripropylsilyl)phenyl)phosphaneyl)-N-(tetrahydro-2H-pyran-4-yl)-1,1-bis(4-(tripropylsilyl)phenyl)phosphanamine)
¹H NMR (500 MHz, C₆D₆) : δ 8.00-7.25 (m, 16H), 3.84-3.76 (m, 2H), 3.60(q, 1H), 2.58-2.48(m, 2H), 1.40-1.30(m, 28H), 1.01-0.92(m, 36H), 0.78-0.71(m, 24H)

### Synthetic Example 7: Synthesis of ligand compound represented by Formula 3-1

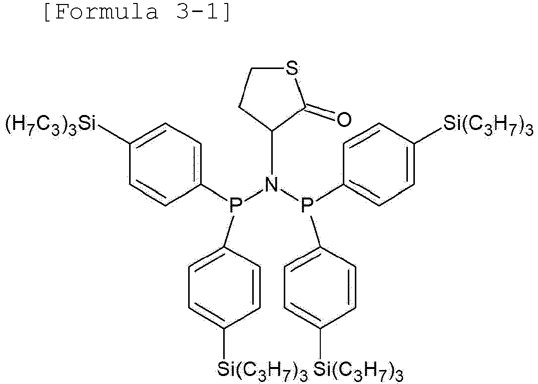

20 mmol (2 eq) of 4-(tripropylsilyl)phenyl bromide was dissolved in 20 ml of tetrahydrofuran and cooled to -78°C. While maintaining the temperature, 20 mmol (2 eq) of n-butyllithium was added thereto dropwisely, followed by stirring for 3 hours. Then, 10 mmol (1 eq) of dichloro(diethylamino)phosphine dissolved in 10 ml of tetrahydrofuran was added thereto dropwisely, and the temperature was raised to room temperature, followed by stirring overnight. Then, the solvent was removed using vacuum, and an intermediate obtained without additional purification was dissolved in 30 ml of hexane, and HCl (in ether, 2 eq) was added thereto. The resultant was subjected to stirring for 15 minutes and filtering. 2.1 mmol (2.1 eq) of an intermediate obtained by drying the filtrate in vacuum was dissolved in 3.8 ml of dichloromethane, and 3 mmol (3 eq) of triethylamine was added thereto. Then, 1 mmol (1 eq) of 2-oxotetrahydrothiophen-3-yl amine dissolved in 3.8 ml of dichloromethane was slowly added to a reaction system, followed by stirring overnight at room temperature. Then, the solvent was removed using vacuum, and the resultant was dissolved in 7.6 ml of hexane and loaded on the top of silica. Silica filtration was performed using hexane with 1 vol% of triethylamine, and the solution thus obtained was concentrated to obtain the ligand compound represented by Formula 3-1.

### <Ligand compound represented by Formula 3-1>

3-(bis(bis(4-(tripropylsilyl)phenyl)phosphaneyl)amino)dihydrothiophen-2(3H)-one
¹H NMR (500 MHz, C₆D₆) : δ 8.00-7.20 (m, 16H), 4.70(t, 1H), 3.05-2.95(m, 2H), 2.43-2.31(m, 2H), 1.40-1.28(m, 24H), 1.10-0.87(m, 36H), 0.88-0.75(m, 24H)

### Synthetic Example 8: Synthesis of ligand compound represented by Formula 4-1

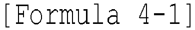

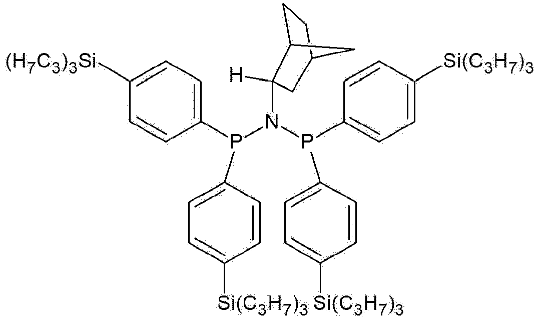

20 mmol (2 eq) of 4-(tripropylsilyl)phenyl bromide was dissolved in 20 ml of tetrahydrofuran and cooled to -78°C. While maintaining the temperature, 20 mmol (2 eq) of n-butyllithium was added thereto dropwisely, followed by stirring for 3 hours. Then, 10 mmol (1 eq) of dichloro(diethylamino)phosphine dissolved in 10 ml of tetrahydrofuran was added thereto dropwisely, and the temperature was raised to room temperature, followed by stirring overnight. Then, the solvent was removed using vacuum, and an intermediate obtained without additional purification was dissolved in 30 ml of hexane, and HCl (in ether, 2 eq) was added thereto. The resultant was subjected to stirring for 15 minutes and filtering. 2.1 mmol (2.1 eq) of an intermediate obtained by drying the filtrate in vacuum was dissolved in 3.8 ml of dichloromethane, and 3 mmol (3 eq) of triethylamine was added thereto. Then, 1 mmol (1 eq) of (1R, 2R, 4S)-bicyclo[2.2.1]heptan-2-yl amine dissolved in 3.8 ml of dichloromethane was slowly added to a reaction system, followed by stirring overnight at room temperature. Then, the solvent was removed using vacuum, and the resultant was dissolved in 7.6 ml of hexane and loaded on the top of silica. Silica filtration was performed using hexane with 1 vol% of triethylamine, and the solution thus obtained was concentrated to obtain the ligand compound represented by Formula 4-1.

### <Ligand compound represented by Formula 4-1>

N-((1R, 2R, 4S)-bicyclo[2.2.1]heptan-2-yl)-N-(bis(4-tripropylsilyl)phenyl)phosphaneyl)-1,1-bis(4-(tripropylsilyl)phenyl)phosphanamine)
¹H NMR (500 MHz, C₆D₆) : δ 7.96-7.66 (m, 4H), 7.64-7.25 (m, 12H), 3.65-3.53(m, 1H), 2.92(d, 1H), 2.53-2.45(m, 1H), 2.27(s, 1H), 1.78(t, 1H), 1.64(s, 1H), 1.45-1.20(m, 24H), 1.05-0.85(m, 41H), 0.82-0.60(m, 24H)

### Synthetic Example 9: Synthesis of ligand compound represented by Formula 2-6

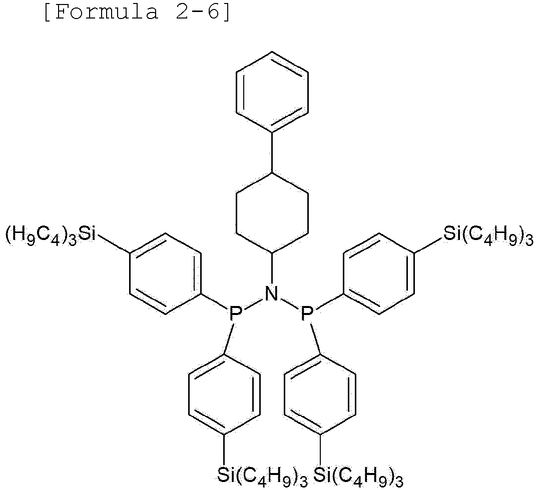

20 mmol (2 eq) of 3-(tributylsilyl)phenyl bromide was dissolved in 20 ml of tetrahydrofuran and cooled to -78°C. While maintaining the temperature, 20 mmol (2 eq) of n-butyllithium was added thereto dropwisely, followed by stirring for 3 hours. Then, 10 mmol (1 eq) of dichloro(diethylamino)phosphine dissolved in 10 ml of tetrahydrofuran was added thereto dropwisely, and the temperature was raised to room temperature, followed by stirring overnight. Then, the solvent was removed using vacuum, and an intermediate obtained without additional purification was dissolved in 30 ml of hexane, and HCl (in ether, 2 eq) was added thereto. The resultant was subjected to stirring for 15 minutes and filtering. 2.1 mmol (2.1 eq) of an intermediate obtained by drying the filtrate in vacuum was dissolved in 3.8 ml of dichloromethane, and 3 mmol (3 eq) of triethylamine was added thereto. Then, 1 mmol (1 eq) of 4-phenylcyclohexylamine dissolved in 3.8 ml of dichloromethane was slowly added to a reaction system, followed by stirring overnight at room temperature. Then, the solvent was removed using vacuum, and the resultant was dissolved in 7.6 ml of hexane and loaded on the top of silica. Silica filtration was performed using hexane with 1 vol% of triethylamine, and the solution thus obtained was concentrated to obtain the ligand compound represented by Formula 2-6.

### <Ligand compound represented by Formula 2-6>

N-(bis(4-(tributylsilyl)phenyl)phosphaneyl)-N-(4-phenylcyclohexyl)-1,1-bis(4-(tributylsilyl)phenyl)phosphanamine)
¹H NMR (500 MHz, C₆D₆) : δ 7.85-6.90 (m, 21H), 3.63-3.45 (m, 0.88H), 3.38-3.27(m, 0.12H), 2.79-2.24(m, 4H), 2.08-1.45(m, 5H), 1.45-1.27(m, 48H), 1.01-0.92(m, 36H), 0.78-0.70(m, 24H)

### Synthetic Example 10: Synthesis of ligand compound represented by Formula 2-4

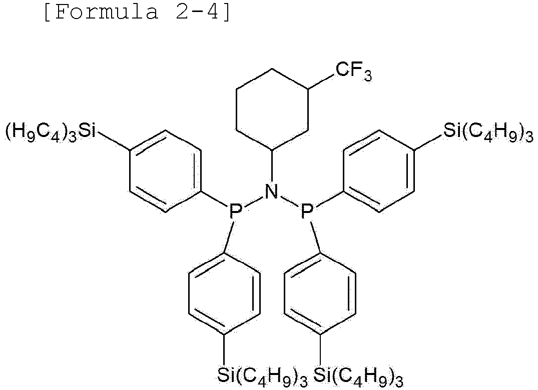

20 mmol (2 eq) of 3-(tributylsilyl)phenyl bromide was dissolved in 20 ml of tetrahydrofuran and cooled to -78°C. While maintaining the temperature, 20 mmol (2 eq) of n-butyllithium was added thereto dropwisely, followed by stirring for 3 hours. Then, 10 mmol (1 eq) of dichloro(diethylamino)phosphine dissolved in 10 ml of tetrahydrofuran was added thereto dropwisely, and the temperature was raised to room temperature, followed by stirring overnight. Then, the solvent was removed using vacuum, and an intermediate obtained without additional purification was dissolved in 30 ml of hexane, and HCl (in ether, 2 eq) was added thereto. The resultant was subjected to stirring for 15 minutes and filtering. 2.1 mmol (2.1 eq) of an intermediate obtained by drying the filtrate in vacuum was dissolved in 3.8 ml of dichloromethane, and 3 mmol (3 eq) of triethylamine was added thereto. Then, 1 mmol (1 eq) of 3-(trifluoromethyl)cyclohexyl amine dissolved in 3.8 ml of dichloromethane was slowly added to a reaction system, followed by stirring overnight at room temperature. Then, the solvent was removed using vacuum, and the resultant was dissolved in 7.6 ml of hexane and loaded on the top of silica. Silica filtration was performed using hexane with 1 vol% of triethylamine, and the solution thus obtained was concentrated to obtain the ligand compound represented by Formula 2-4.

### <Ligand compound represented by Formula 2-4>

N-(bis(4-(tributylsilyl)phenyl)phosphaneyl)-1,1-bis(4-(tributylsilyl)phenyl)-N-(3-(trifluoromethyl)cyclohexyl)phosphanamine)
¹H NMR (500 MHz, C₆D₆) : δ 8.00-7.15 (m, 16H), 3.94-3.80 (m, 0.18H), 3.30(pent, 0.82H), 2.18-1.97(m, 2H), 1.68-1.53(m, 2H), 1.50-1.21(m, 51H), 1.05-0.90(m, 36H), 0.79-0.70(m, 24H)

### Synthetic Example 11: Synthesis of ligand compound represented by Formula 2-7

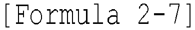

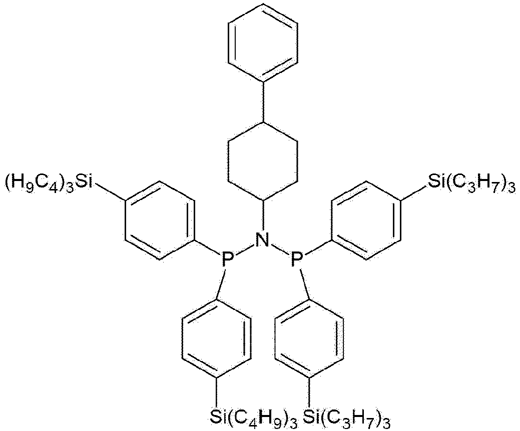

To a dried flask, 11 mmol (2.2 eq) of 4-phenylcyclohexylamine and 10 ml of dichloromethane were injected under a nitrogen atmosphere and subjected to stirring. To the flask, 5 mmol (1 eq) of chlorobis(4-(tripropylsilyl)phenyl)phosphane in a diluted state in 10 ml of dichloromethane was slowly injected. The reaction mixture was stirred for 4 hours, and the conversion was confirmed through ¹H NMR. After finishing the reaction, a precipitated solid was removed through a filter, the solvent was removed under a reduced pressure, and through column chromatography, 5 mmol of an intermediate compound of N-(4-phenylcyclohexyl)-1,1-bis(4-(tripropylsilyl)phenyl)phosphanamine was obtained.

Then, to a dried flask, 5 mmol (1 eq) of the intermediate compound of N-(4-phenylcyclohexyl)-1,1-bis(4-(tripropylsilyl)phenyl)phosphanamine and 13 ml of methyl t-butyl ether were injected under a nitrogen atmosphere and cooled to -78°C, followed by stirring. To the flask, 2.1 ml (5.25 mmol, 1.05 eq) of a solution in which n-butyllithium was dissolved in hexane in 2.5 M, was slowly injected and stirred for 1 hour. Then, 5 mmol (1 eq) of chlorobis(4-(tributylsilyl)phenyl)phosphane in a diluted state in 10 ml of methyl tert-butyl ether was slowly injected. The reaction mixture was stirred for 4 hours, and the conversion was confirmed through ¹H NMR. After finishing the reaction, a precipitated solid was removed through a filter, the solvent was removed under a reduced pressure, and through column chromatography, 4 mmol (yield 80%) of the ligand compound represented by Formula 4-1 was obtained.

### <Ligand compound represented by Formula 2-7>

N-(bis(4-(tributylsilyl)phenyl)phosphaneyl)-N-(4-phenylcyclohexyl)-1,1-bis(4-(tripropylsilyl)phenyl)phosphanamine
¹H NMR (500 MHz, C₆D₆) : δ 7.98-6.90 (m, 21H), 3.64-3.46 (m, 0.88H), 3.39-3.28(m, 0.12H), 2.80-2.25(m, 4H), 2.09-1.46(m, 5H), 1.45-1.27(m, 36H), 1.00-0.92(m, 36H), 0.79-0.71(m, 24H)

### Synthetic Example 12: Synthesis of ligand compound represented by Formula 2-14

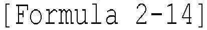

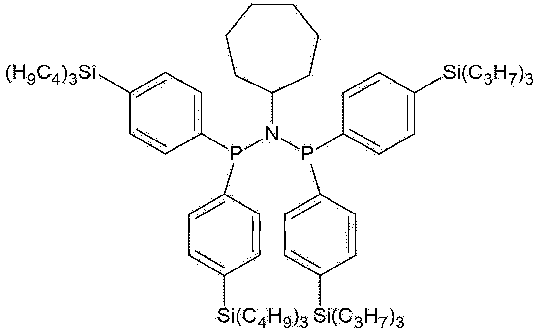

To a dried flask, 11 mmol (2.2 eq) of cycloheptylamine and 10 ml of dichloromethane were injected under a nitrogen atmosphere and subjected to stirring. To the flask, 5 mmol (1 eq) of chlorobis(4-(tripropylsilyl)phenyl)phosphane in a diluted state in 10 ml of dichloromethane was slowly injected. The reaction mixture was stirred for 4 hours, and the conversion was confirmed through ¹H NMR. After finishing the reaction, a precipitated solid was removed through a filter, the solvent was removed under a reduced pressure, and through column chromatography, 5 mmol of an intermediate compound of N-cycloheptyl-1,1-bis(4-(tripropylsilyl)phenyl)phosphanamine was obtained.

Then, to a dried flask, 5 mmol (1 eq) of the intermediate compound of N-cycloheptyl-1,1-bis(4-(tripropylsilyl)phenyl)phosphanamine and 13 ml of methyl t-butyl ether were injected under a nitrogen atmosphere and cooled to -78°C, followed by stirring. To the flask, 2.1 ml (5.25 mmol, 1.05 eq) of a solution in which n-butyllithium was dissolved in hexane in 2.5 M, was slowly injected and stirred for 1 hour. Then, 5 mmol (1 eq) of chlorobis(4-(tributylsilyl)phenyl)phosphane in a diluted state in 10 ml of methyl tert-butyl ether was slowly injected. The reaction mixture was stirred for 4 hours, and the conversion was confirmed through ¹H NMR. After finishing the reaction, a precipitated solid was removed through a filter, the solvent was removed under a reduced pressure, and through column chromatography, 4 mmol (yield 80%) of the ligand compound represented by Formula 4-1 was obtained.

### <Ligand compound represented by Formula 2-14>

N-(bis(4-(tributylsilyl)phenyl)phosphaneyl)-N-cycloheptyl-1,1-bis(4-(tripropylsilyl)phenyl)phosphanamine
¹H NMR (500 MHz, C₆D₆) : δ 8.00-7.15 (m, 16H), 3.63 (pent, 1H), 2.33-2.20(m, 2H), 1.94-1.85(m, 2H), 1.60-1.45(m, 4H), 1.44-1.15(m, 36H), 1.00-0.86(m, 36H), 0.85-0.80(m, 16H), 0.78-0.67(m, 12H)

### Comparative Synthetic Example 1: Synthesis of ligand compound represented by Formula 9

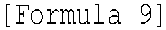

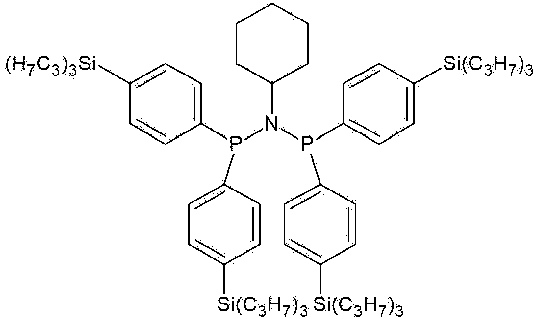

20 mmol (2 eq) of 4-(tripropylsilyl)phenyl bromide was dissolved in 20 ml of tetrahydrofuran and cooled to -78°C. While maintaining the temperature, 20 mmol (2 eq) of n-butyllithium was added thereto dropwisely, followed by stirring for 3 hours. Then, 10 mmol (1 eq) of dichloro(diethylamino)phosphine dissolved in 10 ml of tetrahydrofuran was added thereto dropwisely, and the temperature was raised to room temperature, followed by stirring overnight. Then, the solvent was removed using vacuum, and an intermediate obtained without additional purification was dissolved in 30 ml of hexane, and HCl (in ether, 2 eq) was added thereto. The resultant was subjected to stirring for 15 minutes and filtering. 2.1 mmol (2.1 eq) of an intermediate obtained by drying the filtrate in vacuum was dissolved in 3.8 ml of dichloromethane, and 3 mmol (3 eq) of triethylamine was added thereto. Then, 1 mmol (1 eq) of cyclohexylamine dissolved in 3.8 ml of dichloromethane was slowly added to a reaction system, followed by stirring overnight at room temperature. Then, the solvent was removed using vacuum, and the resultant was dissolved in 7.6 ml of hexane and loaded on the top of silica. Silica filtration was performed using hexane with 1 vol% of triethylamine, and the solution thus obtained was concentrated to obtain the ligand compound represented by Formula 9.

### Comparative Synthetic Example 2: Synthesis of ligand compound represented by Formula 10

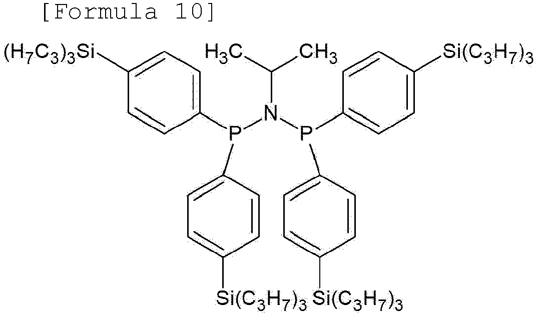

20 mmol (2 eq) of 4-(tripropylsilyl)phenyl bromide was dissolved in 20 ml of tetrahydrofuran and cooled to -78°C. While maintaining the temperature, 20 mmol (2 eq) of n-butyllithium was added thereto dropwisely, followed by stirring for 3 hours. Then, 10 mmol (1 eq) of dichloro(diethylamino)phosphine dissolved in 10 ml of tetrahydrofuran was added thereto dropwisely, and the temperature was raised to room temperature, followed by stirring overnight. Then, the solvent was removed using vacuum, and an intermediate obtained without additional purification was dissolved in 30 ml of hexane, and HCl (in ether, 2 eq) was added thereto. The resultant was subjected to stirring for 15 minutes and filtering. 2.1 mmol (2.1 eq) of an intermediate obtained by drying the filtrate in vacuum was dissolved in 3.8 ml of dichloromethane, and 3 mmol (3 eq) of triethylamine was added thereto. Then, 1 mmol (1 eq) of isopropylamine dissolved in 3.8 ml of dichloromethane was slowly added to a reaction system, followed by stirring overnight at room temperature. Then, the solvent was removed using vacuum, and the resultant was dissolved in 7.6 ml of hexane and loaded on the top of silica. Silica filtration was performed using hexane with 1 vol% of triethylamine, and the solution thus obtained was concentrated to obtain the ligand compound represented by Formula 10.

### Comparative Synthetic Example 3: Synthesis of ligand compound represented by Formula 11

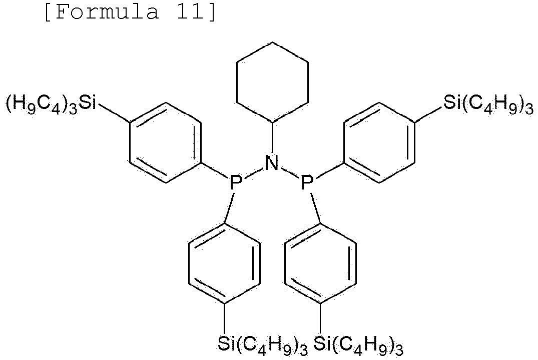

20 mmol (2 eq) of 4-(tributylsilyl)phenyl bromide was dissolved in 20 ml of tetrahydrofuran and cooled to -78°C. While maintaining the temperature, 20 mmol (2 eq) of n-butyllithium was added thereto dropwisely, followed by stirring for 3 hours. Then, 10 mmol (1 eq) of dichloro(diethylamino)phosphine dissolved in 10 ml of tetrahydrofuran was added thereto dropwisely, and the temperature was raised to room temperature, followed by stirring overnight. Then, the solvent was removed using vacuum, and an intermediate obtained without additional purification was dissolved in 30 ml of hexane, and HCl (in ether, 2 eq) was added thereto. The resultant was subjected to stirring for 15 minutes and filtering. 2.1 mmol (2.1 eq) of an intermediate obtained by drying the filtrate in vacuum was dissolved in 3.8 ml of dichloromethane, and 3 mmol (3 eq) of triethylamine was added thereto. Then, 1 mmol (1 eq) of cyclohexylamine dissolved in 3.8 ml of dichloromethane was slowly added to a reaction system, followed by stirring overnight at room temperature. Then, the solvent was removed using vacuum, and the resultant was dissolved in 7.6 ml of hexane and loaded on the top of silica. Silica filtration was performed using hexane with 1 vol% of triethylamine, and the solution thus obtained was concentrated to obtain the ligand compound represented by Formula 11.

### Comparative Synthetic Example 4: Synthesis of ligand compound represented by Formula 12

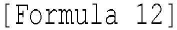

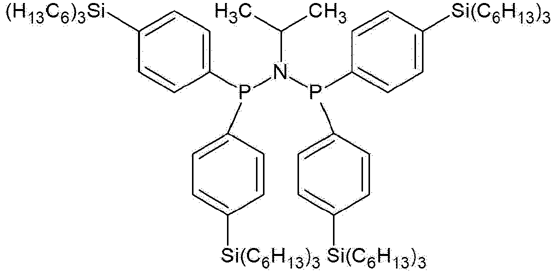

20 mmol (2 eq) of 4-(trihexylsilyl)phenyl bromide was dissolved in 20 ml of tetrahydrofuran and cooled to -78°C. While maintaining the temperature, 20 mmol (2 eq) of n-butyllithium was added thereto dropwisely, followed by stirring for 3 hours. Then, 10 mmol (1 eq) of dichloro(diethylamino)phosphine dissolved in 10 ml of tetrahydrofuran was added thereto dropwisely, and the temperature was raised to room temperature, followed by stirring overnight. Then, the solvent was removed using vacuum, and an intermediate obtained without additional purification was dissolved in 30 ml of hexane, and HCl (in ether, 2 eq) was added thereto. The resultant was subjected to stirring for 15 minutes and filtering. 2.1 mmol (2.1 eq) of an intermediate obtained by drying the filtrate in vacuum was dissolved in 3.8 ml of dichloromethane, and 3 mmol (3 eq) of triethylamine was added thereto. Then, 1 mmol (1 eq) of isopropylamine dissolved in 3.8 ml of dichloromethane was slowly added to a reaction system, followed by stirring overnight at room temperature. Then, the solvent was removed using vacuum, and the resultant was dissolved in 7.6 ml of hexane and loaded on the top of silica. Silica filtration was performed using hexane with 1 vol% of triethylamine, and the solution thus obtained was concentrated to obtain the ligand compound represented by Formula 12.

### Comparative Synthetic Example 5: Synthesis of ligand compound represented by Formula 13

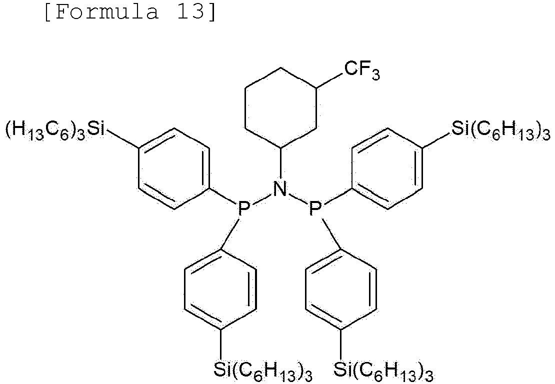

20 mmol (2 eq) of 4-(trihexylsilyl)phenyl bromide was dissolved in 20 ml of tetrahydrofuran and cooled to -78°C. While maintaining the temperature, 20 mmol (2 eq) of n-butyllithium was added thereto dropwisely, followed by stirring for 3 hours. Then, 10 mmol (1 eq) of dichloro(diethylamino)phosphine dissolved in 10 ml of tetrahydrofuran was added thereto dropwisely, and the temperature was raised to room temperature, followed by stirring overnight. Then, the solvent was removed using vacuum, and an intermediate obtained without additional purification was dissolved in 30 ml of hexane, and HCl (in ether, 2 eq) was added thereto. The resultant was subjected to stirring for 15 minutes and filtering. 2.1 mmol (2.1 eq) of an intermediate obtained by drying the filtrate in vacuum was dissolved in 3.8 ml of dichloromethane, and 3 mmol (3 eq) of triethylamine was added thereto. Then, 1 mmol (1 eq) of 3-(trifluoromethyl)cyclohexylamine dissolved in 3.8 ml of dichloromethane was slowly added to a reaction system, followed by stirring overnight at room temperature. Then, the solvent was removed using vacuum, and the resultant was dissolved in 7.6 ml of hexane and loaded on the top of silica. Silica filtration was performed using hexane with 1 vol% of triethylamine, and the solution thus obtained was concentrated to obtain the ligand compound represented by Formula 13.

### Examples and Comparative Examples

### Example 1

Under an argon gas atmosphere, 0.5 mmol of chromium(III) chloride tetrahydrofuran (Cr(THF)₃Cl₃), 0.5 mmol of the ligand compound represented by Formula 2-1 according to Synthetic Example 1, and 0.5 mmol of N,N-dimethylanilinium tetrakis(pentafluorophenyl)borate (AB) as a cocatalyst were put in a flask, and 30 ml of dichloromethane was injected thereto, followed by stirring for 1 hour and removing the solvent in vacuum. Then, the resultant was dissolved in methylcyclohexane and filtered, and the solvent was removed again in vacuum. The resultant was dissolved in methylcyclohexane to prepare a catalyst solution of 0.15 mM (based on Cr).

A Parr reactor with a volume of 600 ml was prepared, vacuum was applied at 120°C for 2 hours, the inside was replaced with argon, and the temperature was reduced to 70°C. Then, 180 ml of methylcyclohexane and 725 pmol of diisobutyl aluminum hydride as 2 ml of an activating agent were injected thereto, and 5 ml (0.75 pmol Cr) of the catalyst solution was injected thereto. After stirring at 1,000 rpm for 2 minutes, the valve of an ethylene line adjusted to 40 bar was opened to fill the inside of the reactor with ethylene, and then, stirring was performed at 1,000 rpm for 60 minutes. The ethylene line valve was closed, the reactor was cooled to 0°C using a dry ice/acetone bath, unreacted ethylene was slowly ventilated, and 0.5 ml of nonane (GC internal standard) was injected. After stirring for 10 seconds, 2 ml of a liquid part of the reactor was taken and quenched with water, and an organic part thus obtained was filtered using a PTFE syringe filter to manufacture a GC-FID sample. Then, the distribution of a liquid product was analyzed by GC (Agilent Co., 6890N, Alltech AT-5 (30 m x 0.32 mm ID x 0.25 um; series no. 12446)). To a remaining solution, 400 ml of ethanol/HCl (10 vol% of aqueous 12 M HCl solution) was added, followed by stirring and filtering. The amount of a solid product was analyzed. The polymer thus obtained was dried in a vacuum oven of 80°C overnight.

### Examples 2 to 12, and Comparative Examples 1 to 5

The same method as in Example 1 was performed except for changing the type of the catalyst as in Table 1 below.

**[Table 1]**

| Division | Ligand compound | Cocatalys t | Chromium source |
|---|---|---|---|
| Example 1 | Synthetic Example 1 (Formula 2-1) | AB | Cr(THF)₃Cl₃ |
| Example 2 | Synthetic Example 2 (Formula 2-3) | AB | Cr(THF)₃Cl₃ |
| Example 3 | Synthetic Example 3 (Formula 2-5) | AB | Cr(THF)₃Cl₃ |
| Example 4 | Synthetic Example 4 (Formula 2-8) | AB | Cr(THF)₃Cl₃ |
| Example 5 | Synthetic Example 5 (Formula 2-10) | AB | Cr(THF)₃Cl₃ |
| Example 6 | Synthetic Example 6 (Formula 2-12) | AB | Cr(THF)₃Cl₃ |
| Example 7 | Synthetic Example 7 (Formula 3-1) | AB | Cr(THF)₃Cl₃ |
| Example 8 | Synthetic Example 8 (Formula 4-1) | AB | Cr(THF)₃Cl₃ |
| Example 9 | Synthetic Example 9 (Formula 2-6) | AB | Cr(THF)₃Cl₃ |
| Example 10 | Synthetic Example 10 (Formula 2-4) | AB | Cr(THF)₃Cl₃ |
| Example 11 | Synthetic Example 11 (Formula 2-7) | AB | Cr(THF)₃Cl₃ |
| Example 12 | Synthetic Example 12 (Formula 2-14) | AB | Cr(THF)₃Cl₃ |
| Comparative Example 1 | Comparative Synthetic Example 1 (Formula 9) | AB | Cr(THF)₃Cl₃ |
| Comparative Example 2 | Comparative Synthetic Example 2 (Formula 10) | AB | Cr(THF)₃Cl₃ |
| Comparative Example 3 | Comparative Synthetic Example 3 (Formula 11) | AB | Cr(THF)₃Cl₃ |
| Comparative Example 4 | Comparative Synthetic Example 4 (Formula 12) | AB | Cr(THF)₃Cl₃ |
| Comparative Example 5 | Comparative Synthetic Example 5 (Formula 13) | AB | Cr(THF)₃Cl₃ |

### Experimental Examples

The results of ethylene oligomerization reaction according to the Examples and Comparative Examples are shown in Table 2 below.
* Catalyst activity (ton/mol·Cr/hr): From the total weight (ton) value of the product obtained by combining the weights (ton) of the liquid product and solid product obtained, the catalyst activity was calculated.
* 1-C6 and 1-C8 selectivity (wt%) : From the GC analysis results of the distribution of a liquid product, the 1-hexene (1-C6) and 1-octene (1-C8) contents were calculated, and the wt% of 1-hexene or 1-octene based on the total weight of the product were calculated.
* Iso-C6, Iso-C8 and C10-C40 production amounts (wt%): From the GC analysis results of the distribution of a liquid product, the contents of iso-hexene (Iso-C6), iso-octene (Iso-C8) and C10-C40 were calculated, and the wt% of iso-hexene (Iso-C6), iso-octene (Iso-C8) and C10-C40 were calculated based on the total weight of the product.
* Solid (wt%): The wt% of the solid product based on the total weight of the product was calculated. The solid means insoluble solids which are not dissolved in a solvent, and represents the degree of production of polyethylene with a carbon number of about 40 or more.

**[Table 2]**

| Division | Catalyst activity | 1-C6 and 1-C8 selectivity | | | Solid |
|---|---|---|---|---|---|
| | | 1-C6 | 1-C8 | Total | |
| | (ton/mol·Cr/hr) | (wt%) | (wt%) | (wt%) | (wt%) |
| Example 1 | 320 | 27.8 | 60.4 | 88.2 | 0.23 |
| Example 2 | 285 | 26.4 | 61.1 | 87.5 | 0.11 |
| Example 3 | 372 | 27.5 | 61.3 | 88.8 | 0.10 |
| Example 4 | 332 | 32.9 | 54.7 | 87.6 | 0.27 |
| Example 5 | 298 | 32.0 | 56.0 | 88.0 | 0.25 |
| Example 6 | 267 | 35.4 | 52.2 | 87.6 | 0.35 |
| Example 7 | 233 | 36.5 | 51.0 | 87.5 | 0.42 |
| Example 8 | 312 | 25.5 | 62.5 | 88.0 | 0.32 |
| Example 9 | 340 | 27.4 | 61.0 | 88.4 | 0.11 |
| Example 10 | 315 | 26.8 | 61.7 | 88.5 | 0.09 |
| Example 11 | 370 | 25.8 | 63.7 | 89.5 | 0.12 |
| Example 12 | 356 | 27.4 | 61.2 | 88.6 | 0.10 |
| Comparative Example 1 | 212 | 24.5 | 60.0 | 84.5 | 1.20 |
| Comparative Example 2 | 170 | 25.7 | 58.9 | 84.6 | 1.10 |
| Comparative Example 3 | 230 | 29.7 | 54.7 | 84.4 | 0.95 |
| Comparative Example 4 | 150 | 27.8 | 56.2 | 84.0 | 2.30 |
| Comparative Example 5 | 210 | 25.1 | 59.0 | 84.1 | 3.54 |

As shown in Table 2, if ethylene oligomerization reaction is performed using the catalyst composition including the ligand compound according to the present invention, it could be confirmed that all the activity, selectivity and stability of the catalyst are improved, because phenyl positioned at the terminal of a diphosphino aminyl moiety has a silyl group substituted with an alkyl group with a specific number as a substituent at the para position, and because of the steric and electronic properties of a substituent substituted at a nitrogen atom.

From the results, it could be confirmed that if ethylene oligomerization is performed using the organochromium compound including the ligand compound and the catalyst composition of the present invention, a linear alpha-olefin may be prepared with excellent productivity due to high catalyst activity, and high selectivity to 1-hexene and 1-octene.

## Claims

1. A ligand compound represented by the following Formula 1: 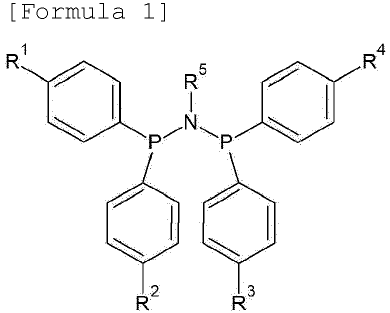
in Formula 1,
R¹ to R⁴ are each independently a trialkylsilyl group, where the alkyl group is an alkyl group of 1 to 4 carbon atoms, and
R⁵ is a substituent represented by any one among the following Formulae 2 to 4: 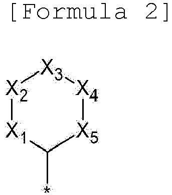 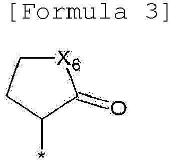 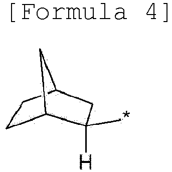
in Formulae 2 to 4,
X₁ to X₆ are each independently - (CR⁶R⁷)ₙ-, -O- or -S-, where all are not -O- or -S- simultaneously, and n is an integer selected from 0 and 1 to 3,
R⁶ and R⁷ are each independently hydrogen, an alkyl group of 1 to 5 carbon atoms, a trifluoroalkyl group of 1 to 5 carbon atoms, an aryl group of 6 to 10 carbon atoms, or -NR⁸R⁹,
R⁸ and R⁹ are each independently hydrogen or an alkyl group of 1 to 5 carbon atoms, and
* is a part bonded to N of Formula 1.

2. The ligand compound according to claim 1, wherein R¹ to R⁴ are each independently a tripropylsilyl group or a tributylsilyl group.

3. The ligand compound according to claim 1, wherein
R⁵ is a substituent represented by any one among Formulae 2 to 4 above,
X₁, X₂ and X₅ are each independently -(CR⁶R⁷)ₙ-,
X₃ and X₄ are each independently -(CR⁶R⁷)ₙ- or -O-, where X₃ and X₄ are not -O- simultaneously,
X₆ is -S-,
R⁶ and R⁷ are each independently hydrogen, a trifluoroalkyl group of 1 to 5 carbon atoms, an aryl group of 6 to 10 carbon atoms, or -NR⁸R⁹, and
R⁸ and R⁹ are each independently hydrogen or an alkyl group of 1 to 5 carbon atoms.

4. The ligand compound according to claim 1, wherein
R⁵ is a substituent represented by Formula 2 above,
X₁ and X₅ are -CH₂-,
X₂ to X₄ are each independently - (CR⁶R⁷)ₙ-, -O- or -S-, where all are not -O- or -S- simultaneously,
R⁶ and R⁷ are each independently hydrogen, a trifluoroalkyl group of 1 to 5 carbon atoms, an aryl group of 6 to 10 carbon atoms, or -NR⁸R⁹, and
R⁸ and R⁹ are each independently hydrogen or an alkyl group of 1 to 3 carbon atoms.

5. The ligand compound according to claim 4, wherein the ligand compound represented by Formula 1 is one selected from the group consisting of the ligand compounds represented by the following Formula 2-1 to Formula 2-14:

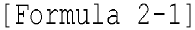

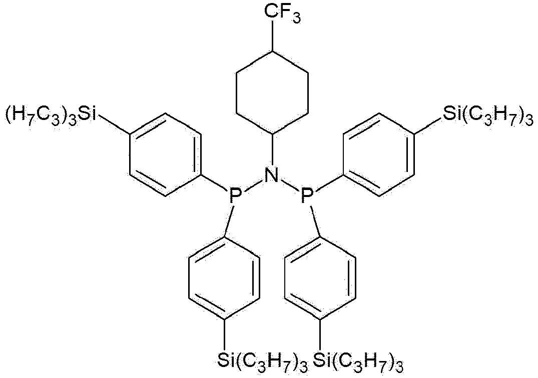

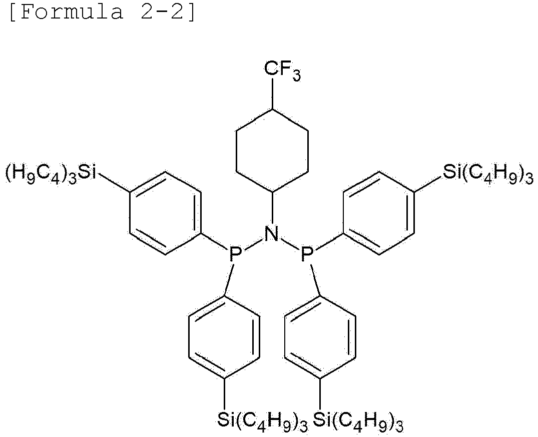

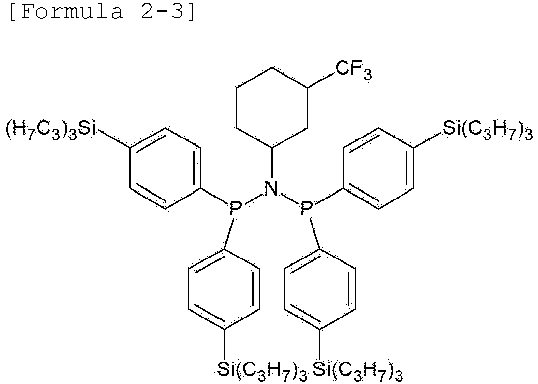

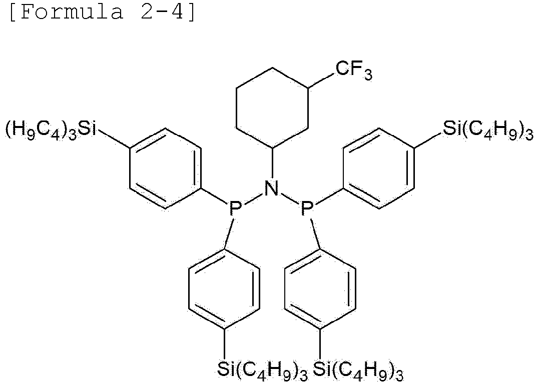

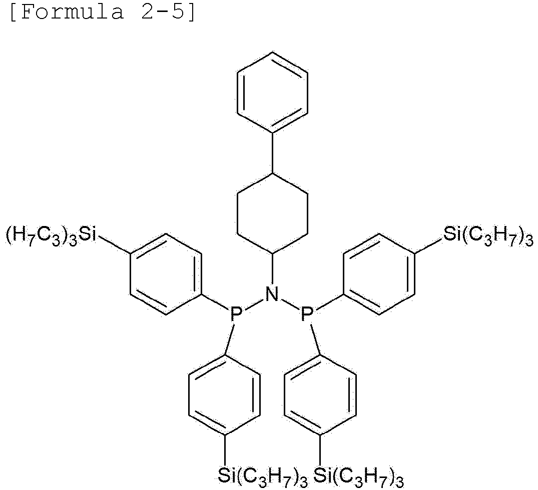

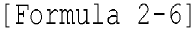

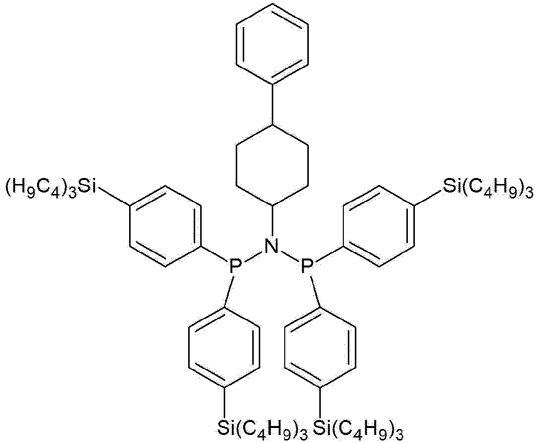

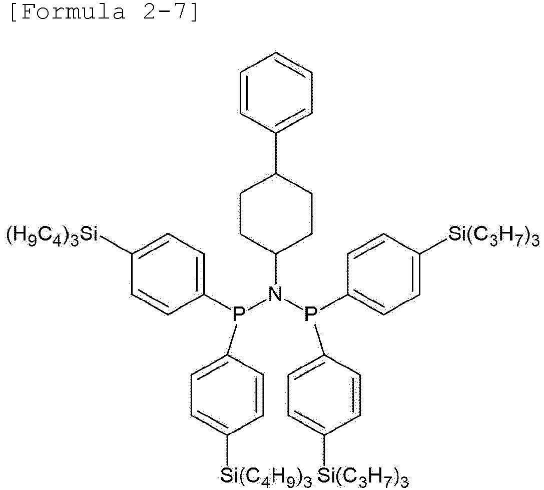

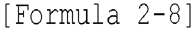

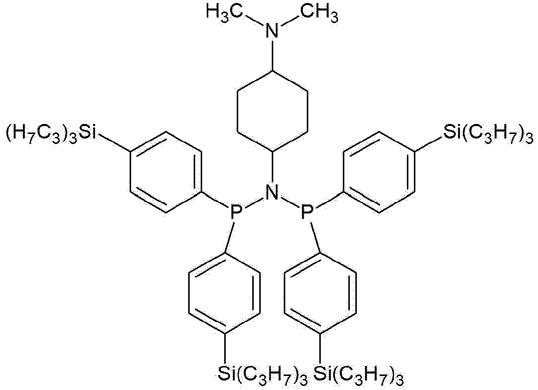

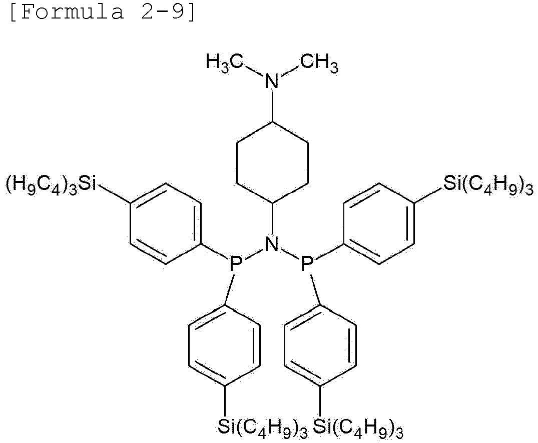

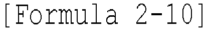

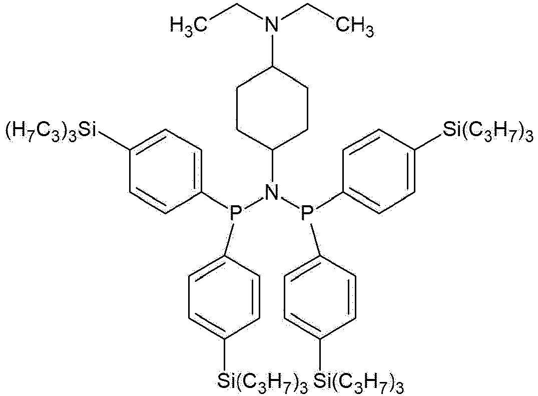

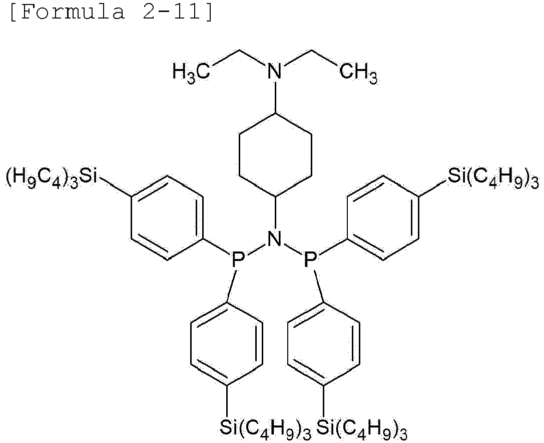

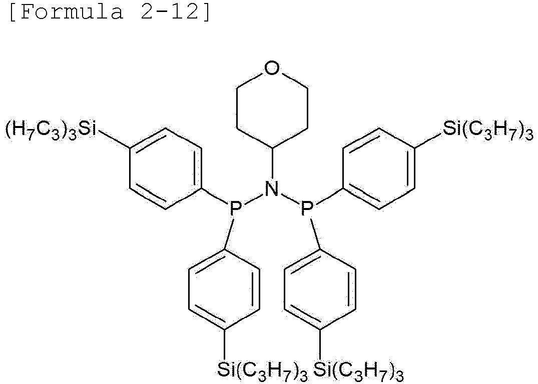

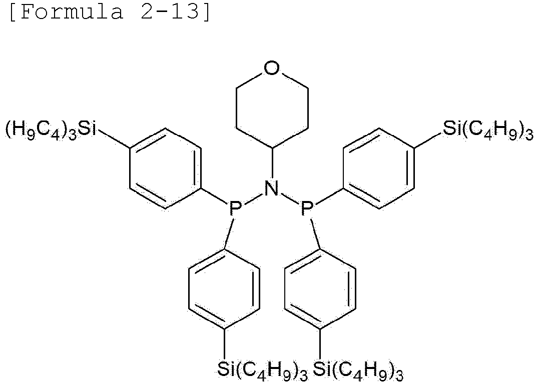

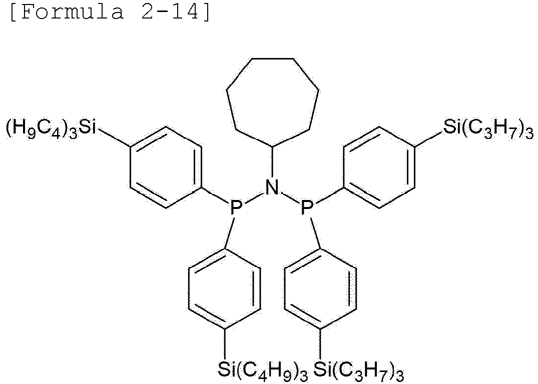

6. The ligand compound according to claim 1, wherein
R⁵ is a substituent represented by Formula 3, and
X₆ is -O- or -S-.

7. The ligand compound according to claim 6, wherein the ligand compound represented by Formula 1 is represented by the following Formula 3-1 or Formula 3-2:

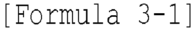

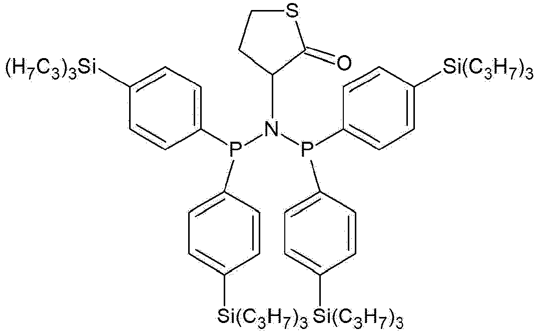

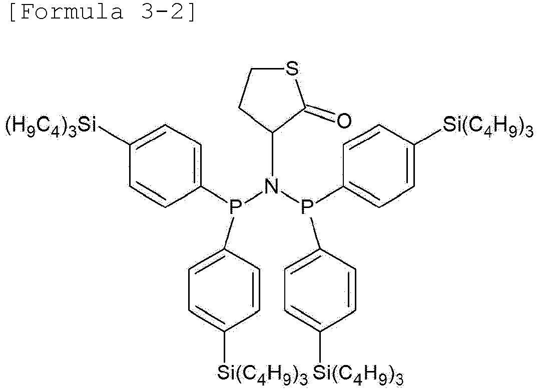

8. The ligand compound according to claim 1, wherein the ligand compound represented by Formula 1 is represented by the following Formula 4-1 or Formula 4-2:

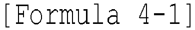

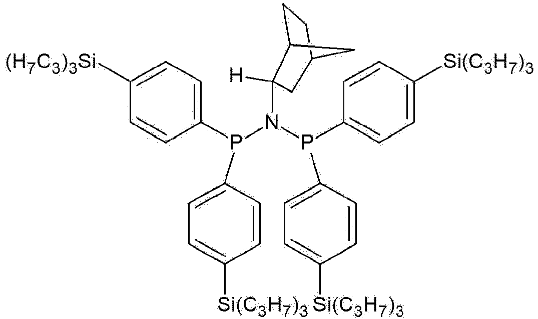

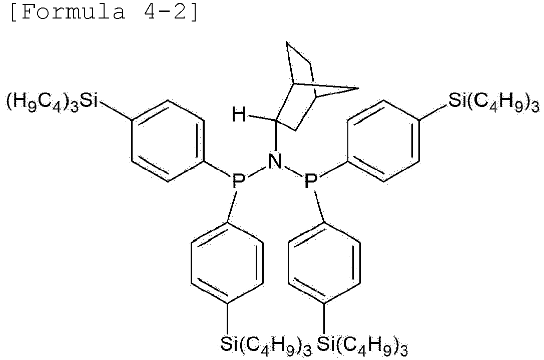

9. An organochromium compound comprising the ligand compound according to claim 1 and chromium making a coordination bond with the ligand compound.

10. The organochromium compound according to claim 9, wherein unshared electron pairs of one or more among N and two P in the ligand compound represented by Formula 1 make coordination bonds with the chromium.

11. A catalyst composition comprising the ligand compound according to claim 1, chromium and a cocatalyst.

12. The catalyst composition according to claim 11, wherein the chromium is derived from a chromium source, and
the chromium source comprises one or more selected from the group consisting of chromium(III) acetylacetonate, chromium(III) chloride tetrahydrofuran, chromium(III) 2-ethylhexanoate, chromium(III) acetate, chromium(III) butyrate, chromium(III) pentanoate, chromium(III) laurate, chromium(III) tris(2,2,6,6-tetramethyl-3,5-heptanedionate) and chromium(III) stearate.

13. The catalyst composition according to claim 11, wherein the cocatalyst is one or more selected from the group consisting of the compounds represented by the following Formula 5 to Formula 8:
[Formula 5] -[Al(R¹⁰)-O]ₐ-
in Formula 5,
each R¹⁰ is independently a halogen group, a hydrocarbyl group of 1 to 20 carbon atoms, or a hydrocarbyl group of 1 to 20 carbon atoms, which is substituted with a halogen group, and
a is an integer of 2 or more,
[Formula 6] E(R¹¹)₃
in Formula 6,
E is aluminum or boron, and
each R¹¹ is independently hydrogen, a halogen group, a hydrocarbyl group of 1 to 20 carbon atoms, or a hydrocarbyl group of 1 to 20 carbon atoms, which is substituted with a halogen group,
[Formula 7] [L-H]⁺[G(Y)₄]⁻
[Formula 8] [L]⁺[G(Y)₄]⁻
in Formula 7 and Formula 8,
L is a neutral or cationic Lewis acid,
[L-H]⁺ is a bronsted acid,
G is an element in group 13, and
each Y is independently a substituted or unsubstituted alkyl group of 1 to 20 carbon atoms or a substituted or unsubstituted aryl group of 6 to 20 carbon atoms, where if the alkyl group or the aryl group is substituted, the substituent is a halogen group, a hydrocarbyl group of 1 to 20 carbon atoms, an alkoxy group of 1 to 20 carbon atoms, or an aryloxy group of 6 to 20 carbon atoms.

14. A method for preparing a linear alpha-olefin, the method comprising a step of oligomerizing ethylene in the presence of the catalyst composition according to claim 11 (S10).

15. The method for preparing a linear alpha-olefin according to claim 14, wherein the linear alpha-olefin is 1-hexene, 1-octene or a mixture of them.
